# EUROPEAN PATENT APPLICATION

(11) **EP 4 425 498 A1**
(43) Date of publication of application: **04.09.2024**
(21) Application number: 22887482.2
(22) Date of filing: 20.10.2022
(51) Int. Cl.: G16B 5/00, G16B 35/00, G16B 40/00, C12M 1/36

(54) **APPARATUS FOR DETERMINING CELL CULTURE CONDITIONS BY USING ARTIFICIAL INTELLIGENCE AND METHOD FOR OPERATING SAME**

(30) Priority: 27.10.2021 KR 20210144620
(71) Applicant: Prestige Biologics Co., Ltd., Cheongju-si, Chungcheongbuk-do 28160 (KR)
(72) Inventor: PARK, Jeong Hoon, Seoul 04968 (KR); SHIM, Jae Ho, Cheongju-si Chungcheongbuk-do 28160 (KR); PARK, Joo Yang, Seoul 04968 (KR)
(74) Representative: Frenkel, Matthias Alexander
(86) International application number: PCT/KR2022/015989
(87) International publication number: WO 2023/075286

(57) **Abstract**

The present invention relates to an apparatus for determining a cell culturing condition and a method of operating the same. The method includes acquiring a first quality attribute and a first influence variable at a first point in time from a cell culture medium, acquiring a first injection variable, applying at least one of the first quality attribute, the first influence variable, and the first injection variable to a culture simulation model and acquiring a second quality attribute and a second influence variable at a second point in time, and selectively changing the first injection variables so that the second quality attribute meets a predetermined condition.

## Description

### BACKGROUND

### 1. Field of the Invention

The present invention relates to an apparatus for determining a cell culturing condition and a method of operating the apparatus, and more particularly, to a method of predicting, by an apparatus for determining a cell culturing condition, changes in influence variables according to injection variables and finding optimal injection variables to acquire desired quality attributes.

### 2. Discussion of Related Art

A bioreactor, which is an apparatus that allows biological reactions or processes to be performed in a laboratory or on an industrial scale, has been widely used within the biopharmaceutical industry. A bioreactor is used to produce various types of bio products. Examples of the bio products may include cell culture media and beverages, bio fuel, bioenergy, biochemical substances, antibiotics, amino acids, enzymes, monoclonal antibodies, multiple antibodies, vaccines, blood products, plasma fraction preparations, proteins, cell culture medicines, cell therapy, gene therapy, tissue engineering products, advanced bio-convergence preparations, food culture media, biopolymers, alcohols, spices, air fresheners, etc.

Meanwhile, the cell culture of microorganisms, plant cells, animal cells, etc. may be performed using methods such as batch culture, fed-batch culture, continuous culture, and perfusion culture. In specific processes, to replenish nutrients contained within a fluid medium or to remove harmful by-products produced during a process, a fluid feed (culture medium) contained within the bioreactor may be removed and resupplied periodically or continuously.

Meanwhile, controlling major metabolites in the fluid feed during the cell culture in the bioreactor may directly affect the quality of products produced. For example, lactate is one of the major metabolites which should be controlled during the growth of cell cultures, especially mammalian cells. For example, a large amount of lactate is produced during a growth phase of cells, whereas when cells enter a stationary phase, the consumed amount of lactate is observed. A high level of lactate may have a negative effect on cell culture. For example, accumulation of lactate may have an effect on product quality attributes. In fact, there is a risk of extreme accumulation of lactate rendering cell cultures commercially unusable.

To this end, a method of controlling the amount of one major metabolite in feed has been proposed. However, in the actual feed, major metabolites affect each other and fluctuate. Therefore, culturing conditions may be optimized only by optimizing all the major metabolites, rather than controlling only specific types of major metabolites.

However, it is very difficult to optimize all the metabolites or balance the metabolites. For example, glucose, glutamine, and glutamate are all variables that have a negative effect on cells when depleted. However, when too much of the glucose, glutamine, and glutamate is injected through a feed, osmolarity may increase and have a negative effect on cells. In addition, the excessive amount of glucose, glutamine, and glutamate itself may have negative effects on cells. In other words, balancing between the metabolites is a fairly complex task.

### SUMMARY OF THE INVENTION

The present invention provides a virtual bioreactor simulation apparatus implemented by performing machine learning that reflects interaction between variables (influence variables or injection variables) affecting cells as much as possible in order to achieve desired quality attributes of the cells.

In addition, the present invention is to derive optimal cell culturing conditions and quality attributes by predicting and analyzing future values of important variables from current variable values (influence variables or injection variables).

According to an embodiment of the present disclosure, a method of operating an apparatus for determining a cell culturing condition includes: acquiring a first quality attribute and a first influence variable at a first point in time from a cell culture medium; acquiring a first injection variable; applying at least one of the first quality attribute, the first influence variable, and the first injection variable to a culture simulation model and acquiring a second quality attribute and a second influence variable at a second point in time; and selectively changing the first injection variables so that the second quality attribute meets a predetermined condition, in which the second point in time is a future of the first point in time.

The first quality attribute may include a first viable cell density (VCD) value and a first amount of immunoglobulin G (IgG).

The first influence variable may include at least one of a first amount of glutamine, a first amount of glutamate, a first amount of glucose, a first amount of lactate, a first amount of ammonia, and first osmolarity.

The acquiring of the first quality attribute and the first influence variable at the first point in time from the cell culture medium may include acquiring a plurality of measured first influence variables at the same point in time, and the acquiring of the second quality attribute and the second influence variable at the second point in time may include acquiring a predicted second influence variable based on the plurality of measured first influence variables.

The first injection variable may include at least one of a first amount of nutrient feed, a first amount of glucose in the nutrient medium, a first amount of oxygen molecules, a first amount of carbon dioxide molecules, a first amount of nitrogen molecules, a first amount of air, a first pH value, a first agitator speed, a first vessel temperature, a first media injection schedule, and a first amount of antifoam.

The selectively changing of the first injection variable may include: within a first period, changing the first injection variable so that a second VCD value included in the second quality attribute has a maximum value; and within a second period, changing the first injection variable so that a second amount of IgG included in the second quality attribute has the maximum value, and the second period may be after the first period.

The selectively changing of the first injection variable may include: within the first period, changing the first injection variable so that a value obtained by multiplying the second VCD value included in the second quality attribute by e^ (-k* second amount of ammonia) has the maximum value; and within the second period, changing the first injection variable so that a value obtained by multiplying the second amount of IgG included in the second quality attribute by e^ (-k* second amount of ammonia) has the maximum value, and k may be a positive number.

The selectively changing of the first injection variable may include: determining whether a second amount of lactate included in the second influence variable is less than or equal to a first threshold amount; when the second amount of lactate is less than or equal to a first threshold amount and within the first period, changing the first injection variables so that the second VCD value included in the second quality attribute has the maximum value; and when the second amount of lactate is less than or equal to the first threshold amount and within the second period, changing the first injection variable so that the second amount of IgG included in the second quality attribute has the maximum value.

The selectively changing of the first injection variable may include: determining whether a second amount of glutamate included in the second influence variable is greater than or equal to a second threshold amount and less than or equal to a third threshold amount; when the second amount of glutamate is greater than or equal to the second threshold amount and less than or equal to the third threshold amount and within the first period, changing the first injection variables so that the second VCD value included in the second quality attribute has the maximum value; and when the second amount of glutamate is greater than or equal to the second threshold amount and less than or equal to the third threshold amount and within the second period, changing the first injection variable so that the second amount of IgG included in the second quality attribute has the maximum value.

The method of operating an apparatus for determining a cell culturing condition may further include transmitting the changed first injection variable to a bioreactor.

According to another embodiment of the present disclosure, an apparatus for determining a cell culturing condition includes: a processor; and a memory, in which, based on instructions stored in the memory, the processor acquires a first quality attribute and a first influence variable at a first point in time from a cell culture medium, acquires a first injection variable, applies at least one of the first quality attribute, the first influence variable, and the first injection variable to a culture simulation model to acquire a second quality attribute and a second influence variable at a second point in time, and selectively changes the first injection variables so that the second quality attribute meets a predetermined condition, and the second point in time may be a future of the first point in time.

### BRIEF DESCRIPTION OF THE DRAWINGS

FIG. 1 is a block diagram of an apparatus for determining a cell culturing condition according to an embodiment of the present invention.
FIG. 2 is a diagram illustrating the apparatus for determining a cell culturing condition according to the embodiment of the present invention.
FIG. 3 is a flowchart for describing a method of operating an apparatus for determining a cell culturing condition according to an embodiment of the present invention.
FIG. 4 is a flowchart for describing a culture simulation model according to an embodiment of the present invention.
FIG. 5 is a flowchart for describing the culture simulation model according to the embodiment of the present invention.
FIG. 6 is a flowchart for describing the culture simulation model according to the embodiment of the present invention.
FIG. 7 is a block diagram for describing a prediction model according to an embodiment of the present invention.
FIG. 8 is a diagram for describing a predetermined condition according to an embodiment of the present invention.
FIG. 9 is a diagram for describing a predetermined condition according to an embodiment of the present invention.
FIG. 10 is a diagram for describing a predetermined condition according to an embodiment of the present invention.
FIG. 11 is a diagram for describing a predetermined condition according to an embodiment of the present invention.
FIG. 12 is a table illustrating a simulation condition according to an embodiment of the present invention.
FIG. 13 is a simulation result according to an embodiment of the present invention.
FIG. 14 is a simulation result according to an embodiment of the present invention.
FIG. 15 is a simulation result according to an embodiment of the present invention.
FIG. 16 is a simulation result according to an embodiment of the present invention.
FIG. 17 is a simulation result according to an embodiment of the present invention.
FIG. 18 is a block diagram for describing a bioreactor system according to an embodiment of the present invention.
FIG. 19 is a flowchart for generally describing the method of operating the apparatus for determining a cell culturing condition according to the embodiment of the present invention.

### DETAILED DESCRIPTION OF EXEMPLARY EMBODIMENTS

Various advantages and features of disclosed embodiments and methods accomplishing them will become apparent from the following description of embodiments with reference to the accompanying drawings. However, the present invention is not limited to exemplary embodiments disclosed below, but may be implemented in various different forms. These embodiments will be provided only in order to make the invention of the present invention complete and allow those skilled in the art to which the present invention pertains to completely recognize the scope of the present invention.

After terms used in the present specification are briefly described, the present invention will be described in detail.

General terms that are currently widely used are selected as terms used in embodiments in consideration of functions in the present specification but may be changed depending on the intention of those skilled in the art or a judicial precedent, the emergence of a new technique, and the like. In addition, in a specific case, terms may be arbitrarily chosen by the applicant. In this case, the meaning of such terms will be mentioned in detail in a corresponding description portion of the present invention. Therefore, the terms used in exemplary embodiments of the present invention should be defined on the basis of the meaning of the terms and the content throughout the present invention rather than the simple names of the terms.

In the present specification, singular forms include plural forms unless the context clearly indicates otherwise. In addition, plural forms include singular forms unless the context clearly indicates otherwise.

Throughout the specification, unless otherwise specified, "including" any component means that other components may be further included rather than excluding other components.

Also, as used herein, the term "unit" refers to a software or hardware component, and a "unit" performs a certain role. However, a "unit" is not meant to be limited to software or hardware. A "unit" may be stored in a storage medium that can be addressed or may be configured to regenerate one or more processors. Accordingly, as an example, "unit" refers to a component such as a software component, an object-oriented software component, a class component, or a task component, a process, a function, an attribute, a procedure, a subroutine, a segment of program code, a driver, firmware, microcode, a circuit, data, a database, a data structure, a table, an array or a variable. Components and functions provided within a "unit" may be combined into a smaller number of components and "units" or may be further separated into additional components and "units."

According to an embodiment of the present invention, a "unit" may be implemented as a processor and a memory. The term "processor" should be interpreted broadly to include a general purpose processor, a central processing unit (CPU), a microprocessor, a digital signal processor (DSP), a controller, a microcontroller, a state machine, or the like. In some contexts, "processor" may refer to an application specific semiconductor (ASIC), a programmable logic device (PLD), a field programmable gate array (FPGA), or the like. The term "processor" may also mean a combination of processing devices, such as a combination of a DSP and a microprocessor, a combination of a plurality of microprocessors, a combination of one or more microprocessors in combination with a DSP core, or any other such configurations.

The term "memory" should be interpreted broadly to include any electronic component capable of storing electronic information. The term "memory" may mean various types of processor-readable media such as a random access memory (RAM), a read-only memory (ROM), a non-volatile random access memory (NVRAM), a programmable read-only memory (PROM), an erase-programmable read-only memory (EPROM), an electrically erasable PROM (EEPROM), a flash memory, magnetic or optical data storage, and registers. The memory is said to be in electronic communication with a processor when the processor is capable of reading and/or writing information from and/or to the memory. The memory integrated in the processor is in electronic communication with the processor.

Hereinafter, embodiments of the invention will be described in detail with reference to the accompanying drawings so that those skilled in the art to which the present invention pertains may easily practice the present invention. In the drawings, parts irrelevant to the description are omitted in order to clarify the description of the present invention.

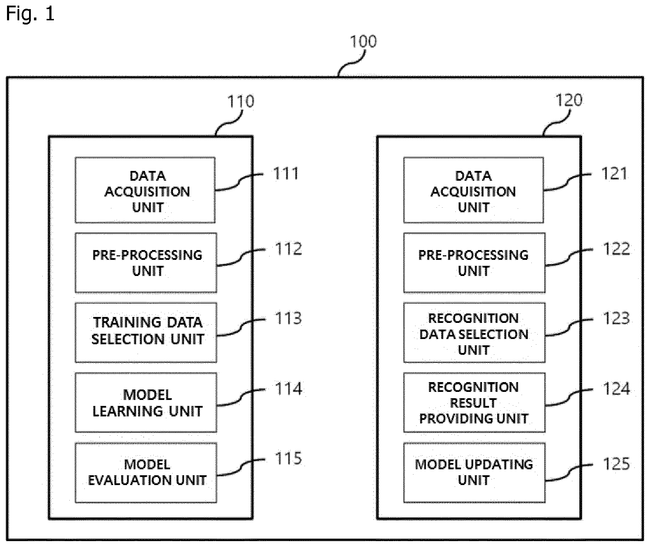

FIG. 1 is a block diagram for describing an apparatus 100 for determining a cell culturing condition according to an embodiment of the present invention.

Referring to FIG. 1, the apparatus 100 for determining a cell culturing condition according to the embodiment may include at least one of a data learning unit 110 and a data recognition unit 120. The apparatus 100 for determining a cell culturing condition as described above may include a processor and a memory. In the present invention, the apparatus 100 for determining a culturing condition may be simply described as the apparatus 100. In addition, the apparatus 100 may include a personal computer (PC), a server, a tablet, a workstation, a smartphone, etc. The apparatus 100 may include an input unit and an output unit. The input unit may be a device for receiving input from a user, and the output unit may be a device for outputting information in the form of sound, video, or data. The apparatus 100 may include a communication unit for transmitting and receiving information to and from an external device. The apparatus 100 may receive information from a user terminal or transmit analysis results to the user terminal.

The data learning unit 110 may train a machine learning model for performing a target task using a data set. The data learning unit 110 may receive label information related to the data set and the target task. The data learning unit 110 may obtain a machine learning model by performing machine learning on a relationship between the data set and the label information. The machine learning model acquired by the data learning unit 110 may be a model for predicting the label information using the data set.

The data recognition unit 120 may receive and store the machine learning model for the data learning unit 110. The data recognition unit 120 may output the predicted label information by applying the machine learning model to input data. In addition, the data recognition unit 120 may use a result output by the input data, the label information, and the machine learning model to update the machine learning model.

At least one of the data learning unit 110 and the data recognition unit 120 may be manufactured in the form of at least one hardware chip and mounted in an electronic device. For example, at least one of the data learning unit 110 and the data recognition unit 120 may be manufactured in the form of a dedicated hardware chip for artificial intelligence (AI) or may be manufactured as a portion of an existing general-purpose processor (e.g., a CPU or an application processor) or a graphics dedicated processor (e.g., a graphics processing unit (GPU)) and mounted in the various electronic devices described above.

In addition, the data learning unit 110 and the data recognition unit 120 may each be mounted in a separate electronic device. For example, one of the data learning unit 110 or the data recognition unit 120 may be included in the electronic device and the other of the data learning unit 110 or the data recognition unit 120 may be included in the server. In addition, the data learning unit 110 and the data recognition unit 120 may provide machine learning model information built by the data learning unit 110 to the data recognition unit 120 or provide data input to the data recognition unit 120 to the data learning unit 110 as additional training data, in a wired or wireless manner.

Meanwhile, at least one of the data learning unit 110 or the data recognition unit 120 may be implemented as a software module. When at least one of the data learning unit 110 and the data recognition unit 120 is implemented as the software module (or a program module including instructions), the software module may be stored in a memory or a non-transitory computer-readable medium. In addition, in this case, at least one software module may be provided by an operating system (OS) or may be provided by a predetermined application. Alternatively, some of one or more software modules may be provided by an OS, and the other software modules may be provided by a predetermined application.

The data learning unit 110 according to the embodiment of the present invention may include a data acquisition unit 111, a pre-processing unit 112, a training data selection unit 113, a model learning unit 114, and a model evaluation unit 115.

The data acquisition unit 111 may obtain data necessary for machine learning. Since much data is required for learning, the data acquisition unit 111 may receive a data set including a plurality of pieces of data.

Label information may be allocated to each of the plurality of pieces of data. The label information may be information describing each of the plurality of pieces of data. The label information may be information to be derived by a target task. The label information may be acquired from a user input, acquired from a memory, or acquired from a result of a machine learning model. The label information may be ground truth information. For example, when the target task is to predict quality attributes or influence variables at a second point in time based on quality attributes, influence variables, and injection variables at the first point in time, the plurality of pieces of data used for the machine learning will be the quality attributes, the influence variables, and the injection variables at the first point in time, and the label information will be the quality attributes or the influence variables at the second point in time. The label information may be the ground truth information, and may be directly input by a user or may be pre-stored in the apparatus 100.

The pre-processing unit 112 may pre-process the obtained data so that the received data may be used for the machine learning. The pre-processing unit 112 may process the acquired data set into a preset format so that a model learning unit 114 to be described later may use the data set.

The training data selection unit 113 may select data necessary for the learning among the pre-processed data. The selected data may be provided to the model learning unit 114. The training data selection unit 113 may select the data necessary for learning among the pre-processed data according to a predetermined criterion. In addition, the training data selection unit 113 may select the data according to a criterion preset by learning by a model learning unit 114 to be described below.

The model learning unit 114 may learn a criterion for which label information to output based on the data set. In addition, the model learning unit 114 may perform machine learning using the data set and the label information on the data set as training data. In addition, the model learning unit 114 may perform machine learning by additionally using a previously acquired machine learning model. In this case, the previously acquired machine learning model may be a model built in advance. For example, the machine learning model may be a model built in advance by receiving basic training data.

The machine learning model may be built in consideration of an application field of the learning model, a purpose of the learning, computer performance of a device, or the like. The machine learning model may be, for example, a model based on a neural network. For example, models such as a deep neural network (DNN), a recurrent neural network (RNN), a long short-term memory model (LSTM), a bidirectional recurrent deep neural network (BRDNN), and a convolutional neural network (CNN) may be used as the machine learning model, but the model is not limited thereto.

According to various embodiments, when a plurality of machine learning models built in advance exist, the model learning unit 114 may determine a machine learning model having a high correlation between input training data and basic training data as a machine learning model to be learned. In this case, the basic training data may be classified in advance for each type of data, and the machine learning model may be built in advance for each type of data. For example, the basic training data may be classified in advance according to various criteria such as a place where the training data is generated, a time at which the training data is generated, a size of the training data, a generator of the training data, and a type of an object in the training data.

In addition, the model learning unit 114 may train the machine learning model using a learning algorithm or the like, including, for example, error back-propagation or gradient descent.

In addition, the model learning unit 114 may train the machine learning model through, for example, supervised learning using the training data as an input value. In addition, the model learning unit 114 may train the machine learning model through, for example, unsupervised learning in which a criterion for a target task is discovered by self-learning a type of data necessary for the target task without special supervision. In addition, the model learning unit 114 may train the machine learning model through, for example, reinforcement learning using feedback on whether a result of the target task according to learning is correct.

In addition, when the machine learning model is trained, the model learning unit 114 may store the trained machine learning model. In this case, the model learning unit 114 may store the trained machine learning model in a memory of an electronic device including the data recognition unit 120. Alternatively, the model learning unit 114 may store the trained machine learning model in a memory of the server connected to the electronic device through a wired or wireless network.

The memory in which the trained machine learning model is stored may also store, for example, commands or data related to at least one other component of the electronic device. In addition, the memory may store software and/or a program. The program may include, for example, a kernel, middleware, an application programming interface (API), an application program (or "application"), and the like.

The model evaluation unit 115 may input evaluation data to the machine learning model, and may cause the model learning unit 114 to train the machine learning model again when a result output from the evaluation data does not meet a predetermined criterion. In this case, the evaluation data may be preset data for evaluating the machine learning model.

For example, the model evaluation unit 115 may evaluate that the trained machined learning model does not meet a predetermined criterion when the number or a ratio of evaluation data for which recognition results are not accurate exceeds a predetermined threshold value among results of the trained machine learning model for the evaluation data. For example, in a case where the predetermined criterion is defined as a ratio of 2%, when the trained machine learning model outputs erroneous recognition results with respect to evaluation data exceeding 20 pieces of evaluation data among a total of 1,000 pieces of evaluation data, the model evaluation unit 115 may evaluate that the trained machined learning model is not appropriate.

Meanwhile, when a plurality of trained machine learning models exist, the model evaluation unit 115 may evaluate whether each of the trained machine learning models meets a predetermined criterion and determine a model meeting the predetermined criterion as a final machine learning model. In this case, when the number of learned machining learning models meeting the predetermined criterion is plural, the model evaluation unit 115 may determine any one preset learned machining learning model or a predetermined number of learned machining learning models as a final machining learning model in order of high evaluation scores.

Meanwhile, at least one of the data acquisition unit 111, the pre-processing unit 112, the training data selection unit 113, the model learning unit 114, and the model evaluation unit 115 within the data learning unit 110 may be manufactured in the form of at least one hardware chip and mounted in the electronic device. For example, at least one of the data acquisition unit 111, the pre-processing unit 112, the training data selection unit 113, the model learning unit 114, and the model evaluation unit 115 may be manufactured in the form of a dedicated hardware chip for AI or may be manufactured as a portion of an existing general-purpose processor (e.g., a CPU or an application processor) or a graphics dedicated processor (e.g., a GPU) and mounted in the various electronic devices described above.

In addition, the data acquisition unit 111, the pre-processing unit 112, the training data selection unit 113, the model learning unit 114, and the model evaluation unit 115 may be mounted in one electronic device or each may be mounted in a separate electronic device. For example, some of the data acquisition unit 111, the pre-processing unit 112, the training data selection unit 113, the model learning unit 114, and the model evaluation unit 115 may be included in the electronic device, and the rest of the data acquisition unit 111, the pre-processing unit 112, the training data selection unit 113, the model learning unit 114, and the model evaluation unit 115 may be included in the server.

In addition, at least one of the data acquisition unit 111, the pre-processing unit 112, the training data selection unit 113, the model learning unit 114, and the model evaluation unit 115 may be implemented as a software module. When at least one of the data acquisition unit 111, the pre-processing unit 112, the training data selection unit 113, the model learning unit 114, and the model evaluation unit 115 is implemented as the software module (or a program module including instructions), the software module may be stored in a non-transitory computer-readable medium. In addition, in this case, at least one software module may be provided by an OS or may be provided by a predetermined application. Alternatively, some of one or more software modules may be provided by an OS, and the other software modules may be provided by a predetermined application.

The data recognition unit 120 according to the embodiment of the present invention may include a data acquisition unit 121, a pre-processing unit 122, a recognition data selection unit 123, a recognition result providing unit 124, and a model updating unit 125.

The data acquisition unit 121 may receive input data. The pre-processing unit 122 may pre-process the acquired input data so that the acquired input data may be used in the recognition data selection unit 123 or the recognition result providing unit 124.

The recognition data selection unit 123 may select necessary data of the pre-processed data. The selected data may be provided to the recognition result providing unit 124. The recognition data selection unit 123 may select some or all of the pre-processed data according to a predetermined criterion. In addition, the recognition data selection unit 123 may select the data according to a criterion preset by learning by the model learning unit 114.

The recognition result providing unit 124 may obtain result data by applying the selected data to the machine learning model. The machine learning model may be the machine learning model generated by the model learning unit 114. The recognition result providing unit 124 may output the result data. For example, the recognition result providing unit 124 may receive a user's current biometric information and output the user's psychological information as result data.

The model updating unit 125 may allow the machine learning model to be updated based on evaluation of the recognition result provided by the recognition result providing unit 124. For example, the model updating unit 125 may provide the recognition result provided by the recognition result providing unit 124 to the model learning unit 114 to cause the model learning unit 114 to update the machine learning model.

Meanwhile, at least one of the data acquisition unit 121, the pre-processing unit 122, the recognition data selection unit 123, the recognition result providing unit 124, and the model updating unit 125 in the data recognition unit 120 may be manufactured in the form of at least one hardware chip and mounted in the electronic device. For example, at least one of the data acquisition unit 121, the pre-processing unit 122, the recognition data selection unit 123, the recognition result providing unit 124, and the model updating unit 125 may be manufactured in the form of a dedicated hardware chip for AI or may be manufactured as a portion of an existing general-purpose processor (e.g., a CPU or an application processor) or a graphics dedicated processor (e.g., a GPU) and mounted in the various electronic devices described above.

In addition, the data acquisition unit 121, the pre-processing unit 122, the recognition data selection unit 123, the recognition result providing unit 124, and the model updating unit 125 may be mounted in one electronic device or each may be mounted in a separate electronic device. For example, some of the data acquisition unit 121, the pre-processing unit 122, the recognition data selection unit 123, the recognition result providing unit 124, and the model updating unit 125 may be included in the electronic device, and the rest of the data acquisition unit 121, the pre-processing unit 122, the recognition data selection unit 123, the recognition result providing unit 124, and the model updating unit 125 may be included in the server.

In addition, at least one of the data acquisition unit 121, the pre-processing unit 122, the recognition data selection unit 123, the recognition result providing unit 124, and the model updating unit 125 may be implemented as a software module. When at least one of the data acquisition unit 121, the pre-processing unit 122, the recognition data selection unit 123, the recognition result providing unit 124, and the model updating unit 125 is implemented as the software module (or a program module including instructions), the software module may be stored in a non-transitory computer-readable medium. In addition, in this case, at least one software module may be provided by an OS or may be provided by a predetermined application. Alternatively, some of one or more software modules may be provided by an OS, and the other software modules may be provided by a predetermined application.

Hereinafter, a method and a device that receive and process the training data with the data acquisition unit 111, the pre-processing unit 112, and the training data selection unit 113 of the data learning unit 110 will be described in more detail.

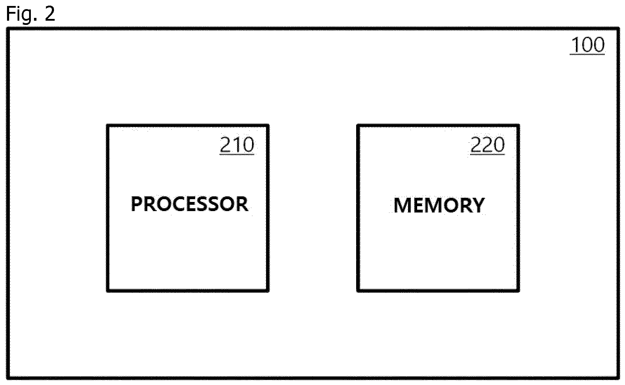

FIG. 2 is a diagram illustrating the apparatus for determining a cell culturing condition according to the embodiment of the present invention.

The apparatus 100 may include a processor 210 and a memory 220. The processor 210 may execute instructions stored in the memory 220.

As described above, the apparatus 100 may include the data learning unit 110 or the data recognition unit 120. The data learning unit 110 or the data recognition unit 120 may be implemented by the processor 210 and the memory 220.

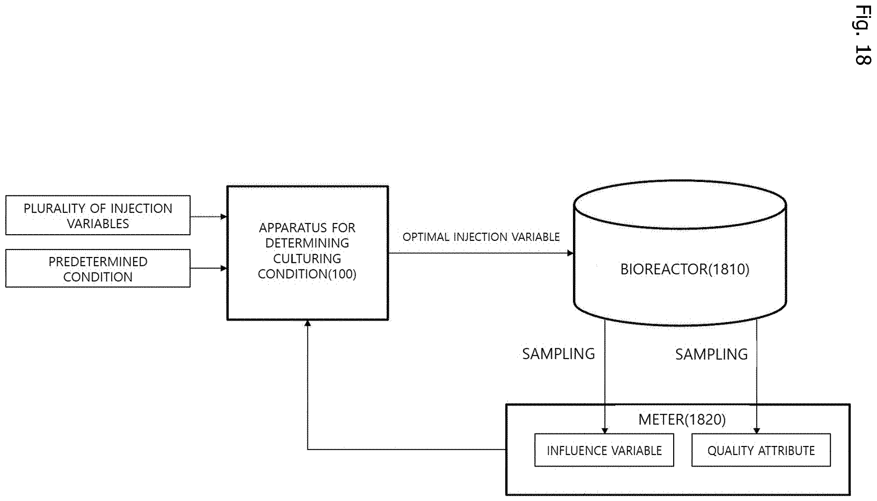

To describe the apparatus 100 in more detail, FIG. 18 will be briefly referenced.

FIG. 18 is a block diagram for describing a bioreactor system according to an embodiment of the present invention.

Referring to FIG. 18, one embodiment of a bioreactor system according to the present invention is illustrated. As illustrated, a cell culture medium is harvested after being cultured in a bioreactor 1810 for a culturing period. Various variables within the bioreactor 1810 are monitored during the culturing. The variables are measured by a meter 1820. According to the present invention, the meter 1820 periodically or continuously monitors the influence variables and quality attributes transmitted to the apparatus 100.

As illustrated in FIG. 18, the apparatus 100 may receive predetermined conditions. The apparatus 100 may select an optimal injection variable from a plurality of injection variables to meet predetermined conditions. The apparatus 100 may include one or more prediction models 730. The prediction model 730 may be generated based on the data learning unit 110 or the data recognition unit 120. The apparatus 100 may include at least one of a classification model or a prediction model.

The classification model may be a model for classifying the influence variables or quality attributes into one of a plurality of classes. The class may be a range of the influence variables or quality attributes. The classification model may predict the probability that the influence variables or quality attributes belong to a specific class. The classification model may use various multivariate methods, including partial least squares analysis alone or linear discriminant analysis as well. In addition, the classification model may use a classification tree, a support vector machine, etc. In one implementation example, a median of a percent probability generated from each classification model may be used as a final percent probability for a cell culture.

The apparatus 100 may also include the prediction model. The prediction model may predict how influence variables and quality attributes will change in the future depending on the injection variables. For example, as illustrated in FIG. 18, the apparatus 100 may acquire the injection variables. The apparatus 100 may predict future influence variables and future quality attributes based on the injection variables. The apparatus 100 may predict the future influence variables and future quality attributes using the prediction model. The future influence variables and future quality attributes may be predicted differently depending on the values of the injection variables. The apparatus 100 may repeatedly perform multiple simulations to determine whether corrective action is needed within the cell culture. That is, the apparatus 100 may repeatedly perform simulations on a plurality of different injection variables. In addition, the apparatus 100 may acquire an optimal injection variable for maximally producing target materials in the bioreactor 1810, and predict the future quality attributes and future influence variables according to the optimal injection variable.

As the future actual influence variables or future actual quality attributes are provided to the apparatus 100, the apparatus 100 may continue to perform simulations throughout the entire culturing period to change one or more conditions within the cell culture medium by further changing or modifying the injection variables.

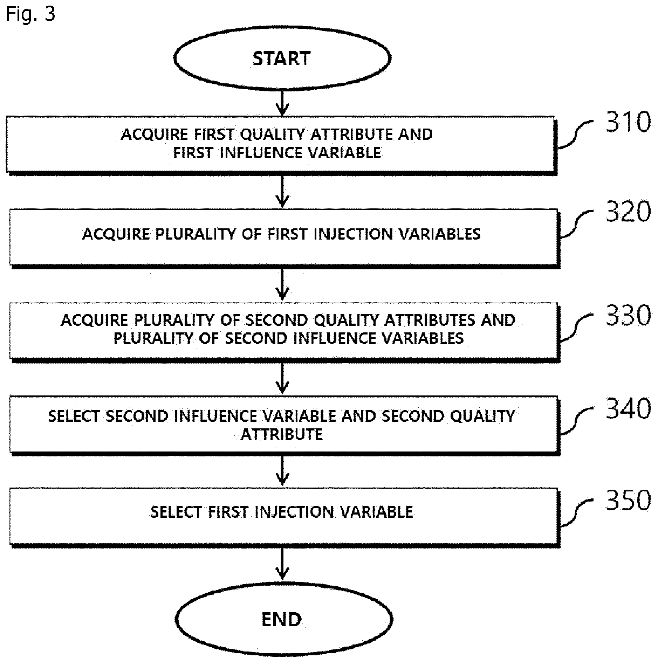

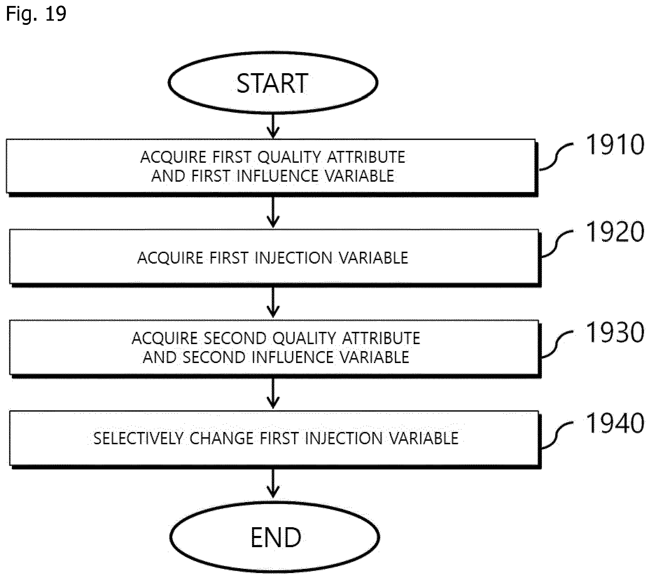

FIG. 3 is a flowchart for describing a method of operating an apparatus for determining a cell culturing condition according to an embodiment of the present invention. In addition, FIG. 19 is a flowchart for generally describing the method of operating the apparatus for determining a cell culturing condition according to the embodiment of the present invention.

First, the operation of the apparatus 100 of the present invention will be generally described with reference to FIG. 19. The apparatus 100 may perform an operation 1910 of acquiring a first quality attribute and a first influence variable at a first point in time from a cell culture medium in order to determine the culturing conditions. The operation 1910 may correspond to an operation 310 of FIG. 3.

The apparatus 100 may perform an operation 1920 of acquiring the first injection variable. The operation 1920 may correspond to an operation 320 of FIG. 3.

The apparatus 100 may perform an operation 1930 of applying at least one of the first quality attribute, the first influence variable, and the first injection variable to a culture simulation model to acquire a second quality attribute and a second influence variable at a second point in time. The operation 1930 may correspond to an operation 330 of FIG. 3.

The second point in time may be a future of the first point in time. The apparatus 100 may perform an operation 1940 of selectively changing the first injection variable so that the second quality attribute meets predetermined conditions. The operation 1940 may correspond to operations 340 and 350 of FIG. 3. That is, the apparatus 100 may perform the operations 340 and 350 to selectively change the first injection variable 1940.

The apparatus 100 may perform a plurality of tests using a plurality of first injection variables in order to selectively change the first injection variable. That is, the apparatus 100 may predict the second quality attribute and the second influence variable for each of the plurality of first injection variables. In addition, the apparatus 100 may select the first injection variable that meets the predetermined condition among the plurality of first injection variables. The predetermined condition may mean that the predicted second quality attribute and the predicted second influence variable are within a specific range. The apparatus 100 may culture cells by modifying the culturing conditions of the cell culture medium using the selected first injection variable. For example, the apparatus 100 may perform the operation of transmitting the changed first injection variable to the bioreactor 1810. The bioreactor 1810 may change the culturing conditions based on the changed first injection variable. Hereinafter, this process will be described in more detail.

The apparatus 100 may perform the operation 310 of acquiring the first quality attribute and the first influence variable from the cell culture medium.

The quality attribute is a value related to a final target that the cell culture medium is intended to reach through the apparatus 100. For example, the quality attribute may include at least one of a viable cell density (VCD) value or the amount of immunoglobulin G (IgG) of the cell culture medium (feed). The VCD value and the amount of IgG may be types of quality attributes. In addition, the quality attributes may also include an antibody titer or antibody quality attributes.

The influence variable may include at least one of the amount of glutamine, the amount of glutamate, the amount of glucose, the amount of lactate, the amount of ammonia, and osmolarity in the feed. The amount of glutamine, the amount of glutamate, the amount of glucose, the amount of lactate, the amount of ammonia, and the osmolarity may be types of influence variables. In this specification, the amount may be the concentration of the corresponding substance in the feed. For example, the units of the amount may be cells/mL, mmol/L, or g/L. However, the unit of the amount is not limited thereto, and the amount in this specification may be an absolute amount of the substance. For example, the unit of the amount may be the number of cells, mol, or g. Also, in this specification, the amount may be the ratio of the substance to the total substance. For example, the unit may be %.

In addition, the first quality attribute and the first influence variable may represent the quality attribute and influence variable of the first point in time. The first point in time may be a point in time that precedes the second point in time. The first point in time may be a point in time that has a measured value. In addition, the second point in time is a future point in time that does not have the measured value, but may be a point in time when a predicted value may be acquired based on the measured value at the first point in time. The second point in time may be after a predetermined time has elapsed from the first point in time. The predetermined time may be 10 minutes, 1 hour, 1 day, etc. In addition, the first point in time is a point in time when the measured value may be measured, and thus may change over time. For example, the first point in time may be the present. However, the first point in time is not limited thereto, and may be in the past or the future. The quality attribute or influence variable of the first point in time may be a measured value or a predicted value in the future. The second point in time is after the predetermined time has passed from the first point in time, and as the first point in time changes, the second point in time may also change.

The first quality attribute may include at least one of a first VCD value and a first amount of IgG. In addition, the first influence variable may include at least one of a first amount of glutamine, a first amount of glutamate, a first amount of glucose, a first amount of lactate, a first amount of ammonia, and first osmolarity.

The apparatus 100 may acquire the first quality attribute and the first influence variable from the user using the input unit included in the apparatus 100. In addition, the apparatus 100 may receive the first quality attribute and the first influence variable from an external device. In addition, the apparatus 100 may measure the first quality attribute and the first influence variable of the feed using a sensor. In addition, the apparatus 100 may acquire the first quality attribute and the first influence variable that are pre-stored in the memory. In addition, the apparatus 100 may acquire the first quality attribute and the first influence variable measured by the meter 1820.

The apparatus 100 may perform an operation of acquiring two or more types of first influence variables measured at the same point in time. That is, the apparatus 100 may measure two or more types of the first amount of glutamine, the first amount of glutamate, the first amount of glucose, the first amount of lactate, the first amount of ammonia, and the first osmolarity. However, the apparatus 100 may acquire one first influence variable without being limited thereto.

The apparatus 100 may perform the operation 320 of acquiring the plurality of first injection variables included within a predetermined range. As described above, the apparatus 100 may perform an operation 1920 of acquiring the first injection variables, but different first injection variables may be used for testing. That is, the apparatus 100 may acquire the plurality of first injection variables to select the optimal first injection variable. The injection variable may be a variable that affects the influence variable and the quality attribute. The injection variable may be a value controlled by the user. A user may control the injection variables to change the influence variables and the quality attributes. The culture simulation model of the present invention may predict future influence variables or quality attributes according to current injection variables. The injection variables may be values related to the substances controlled to culture cells in the feed. The apparatus 100 may receive the injection variables from the user. In addition, the apparatus 100 may receive the injection variables from other apparatuses. In addition, the apparatus 100 may acquire the injection variables from the memory. In addition, the apparatus 100 may acquire the injection variables based on a predetermined algorithm. For example, the apparatus 100 may randomly acquire the injection variables within the predetermined range.

The injection variables or sub-injection variables may include at least one of the amount of nutrient feed, the amount of glucose in the nutrient feed, the amount of oxygen molecules (O₂), the amount of carbon dioxide (CO₂) molecules, the amount of nitrogen molecules (N₂), the amount of air, a pH value, an agitator speed, a vessel temperature, a media injection schedule, and the amount of antifoam. Here, the amount of oxygen molecules may be the amount of dissolved oxygen in the feed.

In addition, the first injection variables may represent injection variables at the first point in time. The first point in time may be a point in time that precedes the second point in time. An interval between the first point in time and the second point in time may be 10 minutes, 1 hour, or 1 day, etc. For example, the first injection variable may include at least one of a first amount of nutrient feed(medium), a first amount of glucose in the nutrient medium, a first amount of oxygen molecules, a first amount of carbon dioxide molecules, a first amount of nitrogen molecules, a first amount of air, a first pH value, a first agitator speed, a first vessel temperature, a first media injection schedule, and a first amount of antifoam.

In the present invention, the "plurality of' first injection variables may mean that there are several sets of first injection variables including at least one of the first amount of nutrient feed, the first amount of glucose in the nutrient medium, the first amount of oxygen molecules, the first amount of carbon dioxide molecules, the first amount of nitrogen molecules, the first amount of air, the first pH value, the first agitator speed, the first vessel temperature, the first media injection schedule, and the first amount of antifoam.

The first injection variable may be included within a predetermined range. The predetermined range may be the range of the value of the first injection variable. The predetermined range may vary depending on the type of first injection variable. The first amount of nutrient feed, the first amount of glucose in the nutrient medium, the first amount of oxygen molecules, the first amount of carbon dioxide molecules, the first amount of nitrogen molecules, the first amount of air, the first pH value, the first agitator speed, the first vessel temperature, the first media injection schedule, and the first amount of antifoam may each be set in a predetermined range. The predetermined range is an experimentally acquired value and may be the range of the injection variables to maximize the quality attributes. The predetermined range may be changed by user settings.

The apparatus 100 may use the plurality of first injection variables to find an optimal injection variable at the first point in time. When the apparatus 100 may perform the operation 320 of acquiring the plurality of first injection variables included within the predetermined range, the apparatus 100 may further perform the following operations. The apparatus 100 may perform an operation of selecting the predetermined number of sub-injection variables among a plurality of sub-injection variables.

The injection variables may include sub-injection variables. The sub-injection variables may be of at least one type included in the injection variables. Alternatively, the injection variables may be a group of sub-injection variables. For example, the sub-injection variables may be each of the amount of nutrient feed, the amount of glucose in the nutrient feed, the amount of oxygen molecules, the amount of carbon dioxide molecules, the amount of nitrogen molecules, the amount of air, the pH value, the agitator speed, the vessel temperature, the media injection schedule, and the amount of antifoam included among the injection variables. The plurality of sub-injection variables may be the amount of nutrient feed, the amount of glucose in the nutrient feed, the amount of oxygen molecules, the amount of carbon dioxide molecules, the amount of nitrogen molecules, the amount of air, the pH value, the agitator speed, the vessel temperature, the media injection schedule, and the amount of antifoam.

The apparatus 100 may select the predetermined number of sub-injection variables. For example, the predetermined number may be two. The apparatus 100 may select the predetermined number of sub-injection variables based on the user's input. Alternatively, the apparatus 100 may automatically select the predetermined number of sub-injection variables. For example, when the predetermined number is two, the selected sub-injection variables may be the pH value and/or the amount of oxygen molecules.

In addition, the apparatus 100 may perform an operation of acquiring candidate values for each selected sub-injection variable. The predetermined range may vary for each sub-injection variable. The apparatus 100 may automatically or manually acquire candidate values for each selected sub-injection variable. The apparatus 100 may select the candidate values for the selected sub-injection variables within the predetermined range and acquire the candidate values for each selected sub-injection variable. More specifically, the apparatus 100 may divide the predetermined range into a plurality of candidate ranges. The apparatus 100 may divide the predetermined range into n equal parts by the number n of predetermined ranges. The number of predetermined ranges may vary for each sub-injection variable. In addition, the apparatus 100 may acquire candidate values by selecting a representative value for each of the plurality of candidate ranges. A representative value of the candidate range may be a median of a candidate range or an average of the minimum and maximum of a candidate range.

For example, the predetermined range of the amount of oxygen molecules (dissolved oxygen in the feed, %), which is the sub-injection variable, may be greater than or equal to 15 and less than 55. The apparatus 100 may determine the plurality of candidate ranges as 15 to 25, 25 to 35, 35 to 45, and 45 to 55. The apparatus 100 may determine 20, 30, 40, and 50, which are medians of the plurality of candidate ranges, as candidate values for the amount (%) of oxygen molecules. Similarly, the predetermined range of pH values, which are the sub-injection variables, may be greater than or equal to 5.5 and less than 8.5. The apparatus 100 may determine the plurality of candidate ranges as 5.5 to 6.5, 6.5 to 7.5, and 7.5 to 8.5. In addition, the apparatus 100 may determine 6, 7, and 8, which are medians of the plurality of candidate ranges, as candidate values for the pH. Similarly, the predetermined range of agitator speed (RPM) may be greater than or equal to 75 and less than or equal to 325. In the same way as above, the apparatus 100 may determine 100, 150, 200, 250, and 300 as candidate values for the agitator speed (RPM).

The apparatus 100 may perform an operation of setting unselected sub-injection variables among the plurality of sub-injection variables to predetermined default values and setting the selected sub-injection variables to one of the candidate values to acquire the plurality of first injection variables. The predetermined default values may vary depending on the type of sub-injection variables. For example, the default value of the vessel temperature may be 36.95 °C.

The apparatus 100 may acquire the plurality of first injection variables based on a combination of candidates for the selected sub-injection variables. For example, when the candidate values for the amount (%) of oxygen molecules are four such as 20, 30, 40, and 50, three candidate values for the pH value are three such as 6, 7, and 8, and three candidate values for the agitator speed (RPM) are five such as 100, 150, 200, 250, and 300, 60 combinations may be generated. That is, the number of first injection variables may be 60. In the above example, three sub-injection variables such as the amount (%) of oxygen molecules, the pH value, and the agitator speed (RPM) may be selected. However, they are not limited thereto. That is, the number of selected sub-inj ection variables may be a number other than 3, and the sub-inj ection variables other than the amount (%) of oxygen molecules, the pH value, and the agitator speed (RPM) may be selected.

The apparatus 100 may perform tests on 60 injection variables and generate 60 predicted influence variables and 60 predicted quality attributes for each of the 60 injection variables. In addition, the apparatus 100 may select the optimal first injection variable with the best quality properties. Here, the test may be the operations 330 to 350. Briefly, the test may be an operation of applying the plurality of first injection variables to the culture simulation model to predict the plurality of second quality attributes and the plurality of second influence variables at the second point in time, and selecting the first injection variable corresponding to the highest value as the optimal first injection variable among the plurality of second quality attributes.

The apparatus 100 may determine the optimal first injection variable with even better quality attributes by performing additional tests on the predetermined range of the optimal first injection variable. For example, the pH value included in the optimal first injection variable may be 7. However, 7 is only a representative value, and there may be an optimal value among values greater than or equal to 6.5 and less than 7.5. The apparatus 100 may divide the range greater than or equal to 6.5 and less than 7.5 into the plurality of candidate ranges and determine the candidate values to determine an extremely optimal first injection variable. The process of determining the extremely optimal first injection variable is the same as the process of determining the optimal first injection variable, and therefore detailed description thereof will be omitted.

Referring to FIG. 3, the apparatus 100 may perform the operation 330 of applying at least one of the first quality attribute, the first influence variable, and the first injection variable to the culture simulation model to acquire the plurality of second quality attributes and the plurality of second influence variables. The culture simulation model may use the prediction results of the plurality of sub-prediction models. The sub-prediction model may be a prediction model that constitutes the culture simulation model. For example, the apparatus 100 may apply the first quality attribute, the first influence variable, and the first injection variable at the first point in time to one sub-prediction model included in the culture simulation model to acquire the second sub-influence variable included in the second influence variable at the second point in time. The apparatus 100 may acquire all the sub-influence variables included in the second influence variable at the second point in time using the plurality of sub-prediction models. In addition, the apparatus 100 may acquire the second quality attribute at the second point in time based on one sub-prediction model included in the culture simulation model.

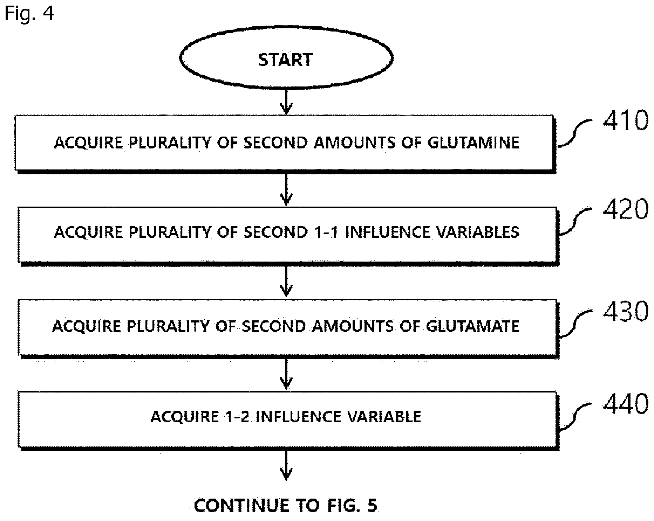

The culture simulation model may be a prediction model formed by combining at least one sub-prediction model. The culture simulation model may include at least one of a prediction model for the changed amount of glutamine, a prediction model for the changed amount of glutamate, a prediction model for the consumed amount of glucose per VCD, a prediction model for the changed amount of lactate, a prediction model for the changed amount of ammonia, a prediction model for osmolarity, a prediction model for a cell division rate, a prediction model for the changed amount of viability, and a prediction model for the amount of IgG produced per VCD. The process of acquiring the plurality of second quality attributes and the plurality of second influence variables using the culture simulation model will be described with reference to FIGS. 4 to 6. The sub-prediction models included in the culture simulation model may be performed in parallel. However, the sub-prediction models are not limited thereto, and the sub-prediction models included in the culture simulation model may be performed in series.

The sub-prediction model included in the culture simulation model may be generated using an ensemble prediction model. The ensemble prediction model may include NGBoost and XGBoost. The machine learning algorithm for generating the sub-prediction model may use a decision-making tree including an NGBoost model or an XGBoost model and linear discriminant analysis. The ensemble prediction model is a method of using a plurality of learning algorithms to obtain better prediction performance compared to using learning algorithms separately. That is, when the ensemble prediction model trains and uses only one machine learning model for one target task (e.g., lactate concentration prediction), since the result value may be unreliable, the ensemble prediction model is a technology that trains other machine learning models on the same target task to derive each result value and then determines a final predicted value based on the result values. For example, in the target task of predicting the lactate concentration, when a model A gives a predicted result of 1 g/L and a model B gives a predicted result of 2 g/L, determining 1.5 g/L, which is the average of these two results, as the final prediction value may be called an ensemble prediction model.

In addition, the ensemble prediction model may include NGBoost and XGBoost based on a gradient boosting machine learning technique. For example, the ensemble prediction model may first include the model A that only broadly trains a target task with a simple structure. In addition, the ensemble prediction model may include the model B that trains more detailed parts than the model A. In addition, the ensemble prediction model may include a model that trains more detailed parts than the model B. The ensemble prediction model may output prediction information for the target task using the model A or the model B.

The sub-prediction models included in the culture simulation model may each be generated using the ensemble prediction model. That is, at least one of the prediction model for the changed amount of glutamine, the prediction model for the changed amount of glutamate, the prediction model for the consumed amount of glucose per VCD, the prediction model for the changed amount of lactate, the prediction model for the changed amount of ammonia, the prediction model for the osmolarity, the prediction model for the cell division rate, the prediction model for the changed amount of viability, and the prediction model for the amount of IgG produced per VCD may be generated using the ensemble prediction model.

Since the apparatus 100 predicts at least one of the influence variable and quality attribute at the second point in time based on the "plurality" of first injection variables, the apparatus 100 may generate at least one of the "plurality" of second influence variables and the "plurality" of second quality attributes. In the present invention, the "plurality of' second quality attributes may mean that there are several sets of the second quality attributes including a second VCD and a second amount of IgG. In addition, in the present invention, the second influence variable may mean that there are several sets of the second influence variables including at least one of a second amount of glutamine, a second amount of glutamate, a second amount of glucose, a second amount of lactate, a second amount of ammonia, and second osmolarity.

The apparatus 100 may perform an operation of acquiring a predicted second influence variable based on two or more measured types of first influence variables. There may be two or more types of influence variables included in the second influence variable. The type of the first influence variable and the type of the second influence variable may be the same. However, the type of the first influence variable and the type of the second influence variable may be different from each other without being limited thereto. In addition, the number of types of the first influence variables and the number of types of the second influence variables may be different from each other.

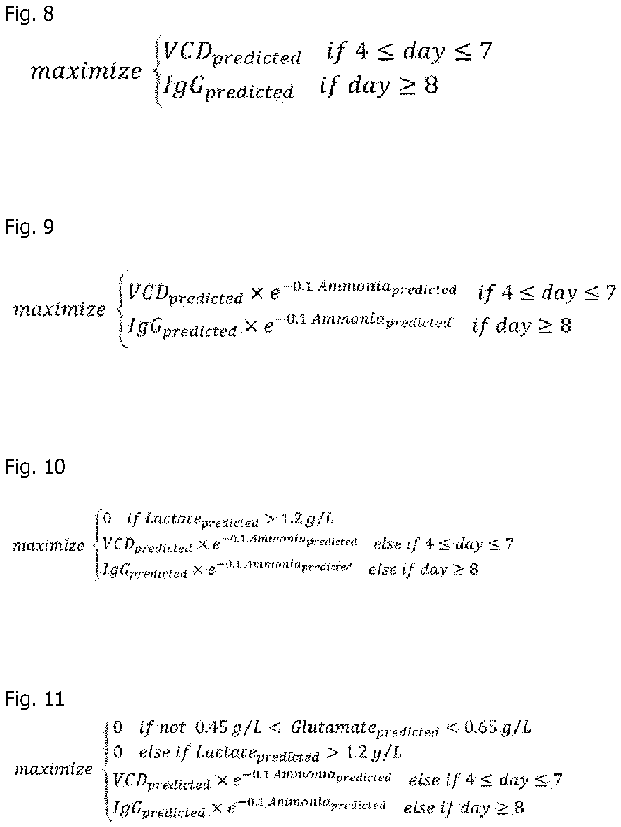

The apparatus 100 may perform the operation 340 of selecting the second influence variable and the second quality attribute that meet the predetermined condition among the plurality of second influence variables and the plurality of second quality attributes. The predetermined conditions will be described with reference to FIGS. 8 to 11.

The apparatus 100 may perform the operation 350 of selecting the first injection variable corresponding to the selected second quality attribute among the plurality of first injection variables. In addition, the apparatus 100 may determine the selected first injection variable as the optimal injection variable. In addition, the apparatus 100 may transmit the optimal injection variable to the bioreactor 1810. In addition, the bioreactor 1810 may determine the culturing conditions for the first point in time based on the received optimal injection variable. In addition, the bioreactor 1810 may compare the measured value of the culture medium and the received optimal injection variable, and control the measured value of the culture medium to reach the optimal injection variables, so that the bioreactor may produce a maximum amount of antibodies.

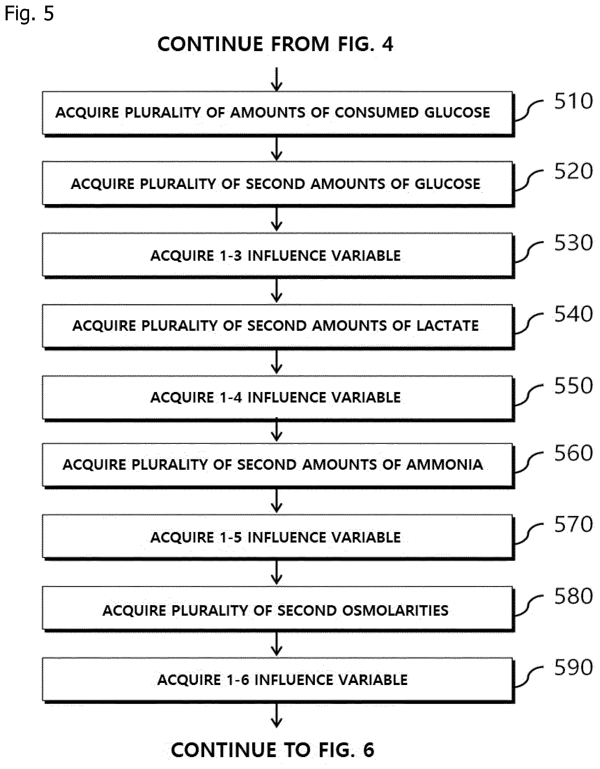

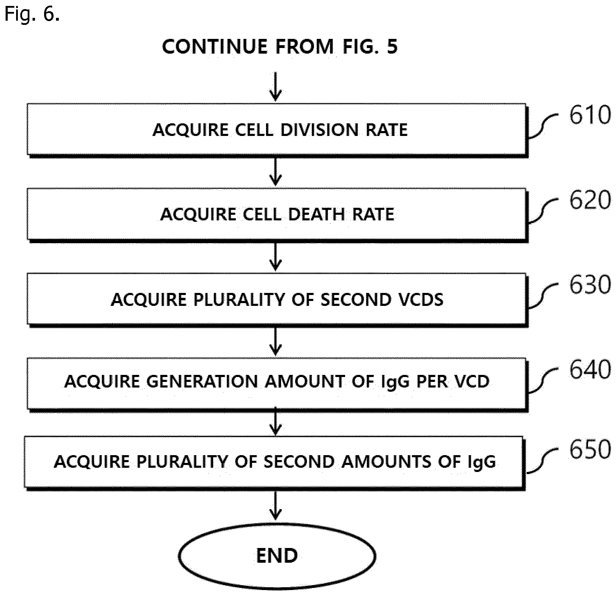

FIG. 4 is a flowchart for describing the culture simulation model according to the embodiment of the present invention. FIG. 5 is a flowchart for describing the culture simulation model according to the embodiment of the present invention. FIG. 6 is a flowchart for describing the culture simulation model according to the embodiment of the present invention.

The operation 330 of acquiring the plurality of second quality attributes and the plurality of second influence variables may include the processes of FIGS. 4 to 6. Referring to FIGS. 4 to 6, the apparatus 100 may include a component that predicts the amount of glutamine and the amount of glutamate, and updates the influence variable based on the predicted amount of glutamine and the predicted amount of glutamate. In addition, the apparatus 100 may include a component that predicts the consumed amount of glucose and updates the influence variable based on the consumed amount of glucose predicted. In addition, the apparatus 100 may include a component that predicts the amount of glucose and updates the influence variable based on the consumed amount of glucose predicted. In addition, the apparatus 100 may include a component that predicts the amount of ammonia and updates the influence variable based on the predicted amount of ammonia. In addition, the apparatus 100 may predict osmolarity and update the influence variable based on the predicted osmolarity. In addition, the apparatus 100 may predict a cell division rate and a cell death rate, and predict the VCD value or the amount of IgG included in the quality attribute based on the predicted cell division rate and cell death rate. Hereinafter, the above configuration will be described in more detail.

Referring to FIG. 4, the apparatus 100 may perform an operation 410 of applying the first quality attribute, the first influence variable, and the plurality of first injection variables to the prediction model for the changed amount of glutamine to acquire the plurality of second amounts of glutamine included in the plurality of second influence variables.

The second influence variable may include at least one of the second amount of glutamine, the second amount of glutamate, the second amount of glucose, the second amount of lactate, the second amount of ammonia, and the second osmolarity. In the present invention, "amount" may be density. For example, the units of the amount may be cells/mL, mmol/L, or g/L. However, the unit of the amount is not limited thereto, and the amount in the present invention may be the absolute amount of the substance. For example, the unit of the amount may be the number of cells, mol, or g. Also, in the present invention, the amount may be the ratio of the substance to the total substance. For example, the unit may be %.

The reason why the plurality of second amounts of glutamine are obtained is to use the plurality of first injection variables. That is, the plurality of first injection variables may correspond one-to-one to the plurality of second amounts of glutamine. As described above, the plurality of first injection variables are combinations generated using the candidate values of the sub-injection variables included in the injection variables. One of the plurality of second amounts of glutamine may be acquired using one of the plurality of first injection variables.

In addition, the apparatus 100 may perform an operation of acquiring a 1-1 influence variable based on one of the plurality of second amounts of glutamine. According to an embodiment of the present invention, the apparatus 100 may perform an operation 420 of acquiring the 1-1 influence variable by replacing the first amount of glutamine included in the first influence variable with one of the plurality of second amounts of glutamine. That is, the apparatus 100 may use the changed first influence variable for operations after the operation 420, and the changed first influence variable may be the 1-1 influence variable.

As described above, the apparatus 100 may generate the plurality of second amounts of glutamine using the plurality of first injection variables. In addition, the apparatus 100 may update the first influence variable using the plurality of second amounts of glutamine. Accordingly, the apparatus 100 may acquire the plurality of 1-1 influence variables that correspond one-to-one to the plurality of second amounts of glutamine. In addition, since the plurality of first injection variables may correspond one-to-one to the plurality of second amounts of glutamine, the plurality of 1-1 influence variables may correspond one-to-one to the plurality of first injection variables. The apparatus 100 may generate the second amount of glutamine using one of the plurality of first injection variables. In addition, the apparatus 100 may acquire the 1-1 influence variable corresponding to one of the plurality of first injection variables. The apparatus 100 may apply, to the sub-prediction model, the 1-1 influence variable corresponding to one of the plurality of first injection variables and one of the plurality of first injection variables in the operations after the operation 420.

In addition, the operation 420 may be replaced by the following operations. That is, the apparatus 100 may perform the operation 420 of acquiring the 1-1 influence variable by replacing the first amount of glutamine included in the first influence variable with the weighted average of the first amount of glutamine and the second amount of glutamine. The weighted average may be equal to w1*(first amount of glutamine)+w2*(second amount of glutamine). Here, w1+w2=1 and w1 and w2 may be greater than or equal to 0 and less than or equal to 1.

The apparatus 100 may perform the operation 430 of applying the first quality attribute, the 1-1 influence variable, and the plurality of first injection variables to the prediction model for the changed amount of glutamate to acquire the plurality of second amounts of glutamate included in the plurality of second influence variables. The reason why the glutamine and glutamate are predicted first in the operations 410 and 430 is because they are variables most directly affected by the nutrient feed injection. When performing the operation 430, the apparatus 100 may replace the 1-1 influence variable with the first influence variable. That is, the apparatus 100 may perform the operation 430 of applying the first quality attribute, the first influence variable, and the plurality of first injection variables to the prediction model for the changed amount of glutamate to acquire the plurality of second amounts of glutamate included in the plurality of second influence variables.

The reason why the plurality of second amounts of glutamate are acquired is to use the plurality of first injection variables. That is, the plurality of first injection variables may correspond one-to-one to the plurality of second amounts of glutamate. One of the plurality of second amounts of glutamate may be acquired using one of the plurality of first injection variables.

In addition, the apparatus 100 may perform an operation of acquiring a 1-2 influence variable based on one of the plurality of second amounts of glutamate. According to an embodiment of the present invention, the apparatus 100 may perform the operation 440 of acquiring the 1-2 influence variable by replacing the first amount of glutamate included in the 1-1 influence variable with one of the plurality of second amounts of glutamate. That is, the apparatus 100 may use the changed first influence variable for operations after the operation 440, and the changed first influence variable may be the 1-2 influence variable.

The apparatus 100 may acquire the plurality of 1-2 influence variables that correspond one-to-one to the plurality of second amounts of glutamate. In addition, the plurality of 1-2 influence variables may correspond one-to-one to the plurality of first injection variables. The apparatus 100 may generate one second amount of glutamate using one of the plurality of first injection variables in the operation 430. In addition, the apparatus 100 may acquire the 1-2 influence variable corresponding to the first injection variable in the operation 440. In the operations after the operation 440, the apparatus 100 may apply the one first injection variable and one 1-2 influence variable corresponding to one of the plurality of 1-2 influence variables to the sub prediction model.

In addition, the operation 440 may be replaced by the following operations. That is, the apparatus 100 may perform the operation 440 of acquiring the 1-2 influence variable by replacing the first amount of glutamine included in the 1-1 influence variable with the weighted average of the first amount of glutamate and the second amount of glutamate. The weighted average may be equal to w3*(first amount of glutamate)+w4*(second amount of glutamate). Here, w3+w4=1 and w3 and w4 may be greater than or equal to 0 and less than or equal to 1.

Referring to FIG. 5, the apparatus 100 may perform an operation 510 of applying the first quality attribute, the 1-2 influence variable, and the plurality of first injection variables to the prediction model for the consumed amount of glucose per VCD to acquire the plurality of amounts of consumed glucose. In addition, the apparatus 100 may perform an operation 520 of acquiring the plurality of second amounts of glucose included in the plurality of second influence variables by subtracting the plurality of amounts of consumed glucose from the first amount of glucose included in a first influence variable. When performing the operation 520, the apparatus 100 may replace the 1-2 influence variable with the first influence variable or the 1-1 influence variable. That is, the apparatus 100 may perform the operation 510 of applying at least one of the first quality attribute, the 1-1 influence variable, the 1-2 influence variable, and the plurality of first injection variables to the prediction model for the consumed amount of glucose per VCD to acquire the plurality of amounts of consumed glucose.

The reason why the plurality of amounts of consumed glucose and the plurality of amounts of glucose are acquired is to use the plurality of first injection variables. That is, the plurality of first injection variables may correspond one-to-one to the plurality of second amounts of consumed glucose. In addition, the plurality of first injection variables may correspond one-to-one to the plurality of amounts of glucose. In addition, one of the plurality of amounts of consumed glucose and one of the plurality of amounts of glucose may be obtained using one of the plurality of first injection variables.

When one of the plurality of amounts of consumed glucose is greater than the first amount of glucose at the first point in time, the apparatus 100 may output a warning when the glucose depletion is expected. Alternatively, the apparatus 100 may remove the first injection variable corresponding to the corresponding amount of consumed glucose from the plurality of first injection variables. That is, the apparatus 100 may not use the first injection variable in which the consumed amount of glucose is predicted to be greater than the first amount of glucose at the first point in time to find the optimal injection variable.

In addition, the apparatus 100 may perform an operation of acquiring a 1-3 influence variable based on one of the plurality of second amounts of glucose. According to an embodiment of the present invention, the apparatus 100 may perform the operation 530 of acquiring the 1-3 influence variable by replacing the first amount of glucose included in the 1-2 influence variable with one of the plurality of amounts of glucose. That is, the apparatus 100 may use the changed first influence variable for operations after the operation 530, and the changed first influence variable may be the 1-3 influence variable.

The apparatus 100 may acquire the plurality of 1-3 influence variables that correspond one-to-one to the plurality of second amounts of glucose. In addition, the plurality of 1-3 influence variables may correspond one-to-one to the plurality of first injection variables. The apparatus 100 may generate one second amount of glucose using one of the plurality of first injection variables in the operation 520. In addition, the apparatus 100 may acquire the 1-3 influence variable corresponding to the first injection variable in the operation 530. In the operations after the operation 530, the apparatus 100 may apply the one first injection variable and one 1-3 influence variable corresponding to one of the plurality of 1-3 influence variables to the sub prediction model.

In addition, the operation 530 may be replaced by the following operations. That is, the apparatus 100 may perform the operation 530 of acquiring the 1-2 influence variable by replacing the first amount of glucose included in the 1-1 influence variable with the weighted average of the first amount of glucose and the second amount of glucose. The weighted average may be equal to w5*(first amount of glucose)+w6*(second amount of glucose). Here, w5+w6=1 and w5 and w6 may be greater than or equal to 0 and less than or equal to 1.

Referring to FIG. 5, the apparatus 100 may perform an operation 540 of applying the first quality attribute, the 1-3 influence variable, and the plurality of first injection variables to the prediction model for the changed amount of lactate to acquire the plurality of second amounts of lactate included in the plurality of second influence variables.

The reason why the plurality of second amounts of lactate are obtained is to use the plurality of first injection variables. That is, the plurality of first injection variables may correspond one-to-one to the plurality of second amounts of lactate. In addition, one of the plurality of second amounts of lactate may be acquired using one of the plurality of first injection variables.

When performing the operation 540, the apparatus 100 may replace the 1-3 influence variable with at least one of the first influence variable, the 1-1 influence variable, or the 1-2 influence variable. That is, the apparatus 100 may perform the operation 540 of applying at least one of the first quality attribute, the first influence variable, the 1-1 influence variable, the 1-2 influence variable, the 1-3 influence variable, and the plurality of first injection variables to the prediction model for the changed amount of lactate to acquire the plurality of second amounts of lactate included in the plurality of second influence variables.

In addition, the apparatus 100 may perform an operation of acquiring a 1-4 influence variable based on one of the plurality of second amounts of lactate. The apparatus 100 may perform an operation 550 of acquiring the 1-4 influence variable by replacing the first amount of lactate included in the 1-3 influence variable with one of the plurality of second amounts of lactate. That is, the apparatus 100 may use the changed first influence variable for operations after the operation 550, and the changed first influence variable may be the 1-4 influence variable.

The apparatus 100 may acquire the plurality of 1-4 influence variables that correspond one-to-one to the plurality of second amounts of lactate. In addition, the plurality of 1-4 influence variables may correspond one-to-one to the plurality of first injection variables. The apparatus 100 may generate one second amount of lactate using one of the plurality of first injection variables in the operation 540. In addition, the apparatus 100 may acquire the 1-4 influence variable corresponding to the first injection variable in the operation 550. In the operations after the operation 550, the apparatus 100 may apply the one first injection variable and one 1-4 influence variable corresponding to one of the plurality of 1-4 influence variables to the sub prediction model.

In addition, the operation 550 may be replaced by the following operations. That is, the apparatus 100 may perform the operation 550 of acquiring the 1-4 influence variable by replacing the first amount of lactate included in the 1-3 influence variable with the weighted average of the first amount of lactate and the second amount of lactate. The weighted average may be equal to w7*(first amount of lactate)+w8*(second amount of lactate). Here, w7+w8=1 and w7 and w8 may be greater than or equal to 0 and less than or equal to 1.

Referring to FIG. 5, the apparatus 100 may perform an operation 560 of applying the first quality attribute, the 1-4 influence variable, and the plurality of first injection variables to the prediction model for the changed amount of ammonia to acquire the plurality of second amounts of ammonia included in the plurality of second influence variables.

The reason why the plurality of second amounts of ammonia are obtained is to use the plurality of first injection variables. That is, the plurality of first injection variables may correspond one-to-one to the plurality of second amounts of ammonia. In addition, one of the plurality of second amounts of ammonia may be acquired using one of the plurality of first injection variables.

When performing the operation 560, the apparatus 100 may replace the 1-4 influence variable with at least one of the first influence variable, the 1-1 influence variable, the 1-2 influence variable, or the 1-3 influence variable. That is, the apparatus 100 may perform the operation 560 of applying at least one of the first quality attribute, the first influence variable, the 1-1 influence variable, the 1-2 influence variable, the 1-3 influence variable, the 1-4 influence variable, and the plurality of first injection variables to the prediction model for the changed amount of ammonia to acquire the plurality of second amounts of ammonia included in the plurality of second influence variables.

In addition, the apparatus 100 may perform an operation of acquiring a 1-5 influence variable based on one of the plurality of second amounts of ammonia. For example, the apparatus 100 may perform an operation 570 of acquiring the 1-5 influence variable by replacing the first amount of ammonia included in the 1-4 influence variable with one of the plurality of second amounts of ammonia. That is, the apparatus 100 may use the changed first influence variable for operations after the operation 570, and the changed first influence variable may be the 1-5 influence variable.

The apparatus 100 may acquire the plurality of 1-5 influence variables that correspond one-to-one to the plurality of second amounts of ammonia. In addition, the plurality of 1-5 influence variables may correspond one-to-one to the plurality of first injection variables. The apparatus 100 may generate one second amount of ammonia using one of the plurality of first injection variables in the operation 560. In addition, the apparatus 100 may acquire the 1-5 influence variable corresponding to the first injection variable in the operation 570. In the operations after the operation 570, the apparatus 100 may apply the one first injection variable and one 1-5 influence variable corresponding to one of the plurality of 1-5 influence variables to the sub prediction model.

In addition, the operation 570 may be replaced by the following operations. That is, the apparatus 100 may perform the operation 570 of acquiring the 1-5 influence variable by replacing the first amount of ammonia included in the 1-4 influence variable with the weighted average of the first amount of ammonia and the second amount of ammonia. The weighted average may be equal to w9*(first amount of ammonia)+w10*(second amount of ammonia). Here, w9+w10=1 and w9 and w10 may be greater than or equal to 0 and less than or equal to 1.

Referring to FIG. 5, the apparatus 100 may perform an operation 580 of applying the first quality attribute, the 1-5 influence variable, and the plurality of first injection variables to the prediction model for osmolarity to acquire the plurality of second osmolarities included in the plurality of second influence variables.

The reason why the plurality of second osmolarities are acquired is to use the plurality of first injection variables. That is, the plurality of first injection variables may correspond one-to-one to the plurality of second osmolarities. In addition, one of the plurality of second osmolarities may be acquired using one of the plurality of first injection variables.

When performing the operation 580, the apparatus 100 may replace the 1-5 influence variable with at least one of the first influence variable, the 1-1 influence variable, the 1-2 influence variable, the 1-3 influence variable, or the 1-4 influence variable. That is, the apparatus 100 may perform the operation 580 of applying at least one of the first quality attribute, the first influence variable, the 1-1 influence variable, the 1-2 influence variable, the 1-3 influence variable, the 1-4 influence variable, the 1-5 influence attribute, and the plurality of first injection variables to the prediction model for osmolarity to acquire the plurality of second osmolarities included in the plurality of second influence variables. In addition, the apparatus 100 may perform an operation of acquiring a 1-6 influence variable based on one of the plurality of second osmolarities. More specifically, the apparatus 100 may perform an operation 590 of acquiring the 1-6 influence variable by replacing the first osmolarity included in the 1-6 influence variable with one of the plurality of second osmolarities. That is, the apparatus 100 may use the changed first influence variable for operations after the operation 590, and the changed first influence variable may be the 1-6 influence variable.

The apparatus 100 may acquire the plurality of 1-6 influence variables that correspond one-to-one to the plurality of second osmolarities. In addition, the plurality of 1-6 influence variables may correspond one-to-one to the plurality of first injection variables. The apparatus 100 may generate one second osmolarity using one of the plurality of first injection variables in the operation 580. In addition, the apparatus 100 may acquire the 1-6 influence variable corresponding to the first injection variable in the operation 590. In the operations after the operation 590, the apparatus 100 may apply the one first injection variable and one 1-6 influence variable corresponding to one of the plurality of 1-6 influence variables to the sub prediction model.

In addition, the operation 590 may be replaced by the following operations. That is, the apparatus 100 may perform the operation 590 of acquiring the 1-6 influence variable by replacing the first osmolarity included in the 1-5 influence variable with the weighted average of the first osmolarity and the second osmolarity. The weighted average may be equal to w11*(first amount of ammonia)+w12*(second amount of ammonia). Here, w11+w12=1 and w11 and w12 may be greater than or equal to 0 and less than or equal to 1.

FIG. 6 illustrates the process of updating the quality attribute using the changed influence variable as described above. The changed influence variable may be the 1-6 influence variable. That is, FIG. 6 illustrates the process of predicting the quality attribute at the second point in time.

Referring to FIG. 6, the apparatus 100 may perform an operation 610 of applying the first quality attribute, the 1-6 influence variable, and the plurality of first injection variables to the prediction model for the cell division rate to acquire the plurality of cell division rates. The cell division rate may be the rate at which cells divide at the first point in time. For example, the division rate of 300% may mean that the number of cells has increased threefold.

When performing the operation 610, the apparatus 100 may replace the 1-6 influence variable with at least one of the first influence variable, the 1-1 influence variable, the 1-2 influence variable, the 1-3 influence variable, the 1-4 influence variable, or the 1-5 influence variable. The apparatus 100 may perform the operation 610 of applying at least one of the first quality attribute, the first influence variable, the 1-1 influence variable, the 1-2 influence variable, the 1-3 influence variable, the 1-4 influence variable, the 1-5 influence variable, the 1-6 influence variable, and the plurality of first injection variables to the prediction model for the cell division rate to acquire the plurality of cell division rates.

The reason why the plurality of cell division rates are acquired is to use the plurality of first injection variables. That is, the plurality of first injection variables may correspond one-to-one to the plurality of cell division rates. In addition, one of the plurality of cell division rates may be acquired using one of the plurality of first injection variables.

The apparatus 100 may perform an operation 620 of applying the first quality attribute, the 1-6 influence variable, and the plurality of first injection variables to the prediction model for the changed amount of viability to acquire the plurality of cell death rates. The cell death rate may be the rate at which cells divide at the first point in time. For example, the cell death rate of 30% may mean that 70% of cells survive. For example, the amount of cells at the second point in time may be equal to (amount of cells at the first point in time) * cell division rate * (100% - cell death rate). The amount of cells at the second point in time may be the second VCD value.

When performing the operation 620, the apparatus 100 may replace the 1-6 influence variable with at least one of the first influence variable, the 1-1 influence variable, the 1-2 influence variable, the 1-3 influence variable, the 1-4 influence variable, or the 1-5 influence variable. The apparatus 100 may perform the operation 620 of applying at least one of the first quality attribute, the first influence variable, the 1-1 influence variable, the 1-2 influence variable, the 1-3 influence variable, the 1-4 influence variable, the 1-5 influence variable, the 1-6 influence variable, and the plurality of first injection variables to the prediction model for the changed amount of viability to acquire the plurality of cell death rates.

The reason why the plurality of cell death rates are acquired is to use the plurality of first injection variables. That is, the plurality of first injection variables may correspond one-to-one to the plurality of cell death rates. In addition, one of the plurality of cell death rates may be acquired using one of the plurality of first injection variables.

The apparatus 100 may perform an operation 630 of acquiring the plurality of second VCD values included in the second plurality of quality attributes based on at least one of a first VCD value included in the first quality attribute, the plurality of cell division rates, and the plurality of cell death rates. The second VCD value may be the predicted VCD value at the second point in time.

The reason why the plurality of second VCD values are acquired is to use the plurality of first injection variables. That is, the plurality of first injection variables may correspond one-to-one to the plurality of second VCD values. In addition, one of the plurality of second VCD values may be acquired using one of the plurality of first injection variables. The apparatus 100 may select the second VCD value that meets the predetermined condition among a plurality of second VCD values. In addition, the apparatus 100 may select the first injection variable corresponding to the selected second VCD value as the optimal first injection variable.

The apparatus 100 may perform an operation 640 of applying the first quality attribute, the 1-6 influence variable, and the plurality of first injection variables to the prediction model of the amount of IgG produced per VCD value to acquire the plurality of amounts of IgG produced per VCD.

When performing the operation 640, the apparatus 100 may replace the 1-6 influence variable with at least one of the first influence variable, the 1-1 influence variable, the 1-2 influence variable, the 1-3 influence variable, the 1-4 influence variable, or the 1-5 influence variable.

The reason why the plurality of amounts of IgG per VCD are acquired is to use the plurality of first injection variables. That is, the plurality of first injection variables may correspond one-to-one to the plurality of amounts of IgG produced per VCD. In addition, one of the plurality of amounts of IgG produced per VCD may be acquired using one of the plurality of first injection variables.

The apparatus 100 may perform an operation 650 of acquiring the plurality of second amounts of IgG included in the plurality of second quality attributes obtained by multiplying the plurality of second VCDs by the amounts of IgG produced per VCD. The apparatus 100 may select a second IgG that meets a predetermined condition, among a plurality of second IgGs. In addition, the apparatus 100 may select the first injection variable corresponding to the selected second VCD value as the optimal first injection variable. In addition, the apparatus 100 may transmit the optimal injection variable to the bioreactor 1810. In addition, the bioreactor 1810 may determine the culturing conditions for the first point in time based on the received optimal injection variable. Through this process, the bioreactor may produce a maximum amount of antibodies.

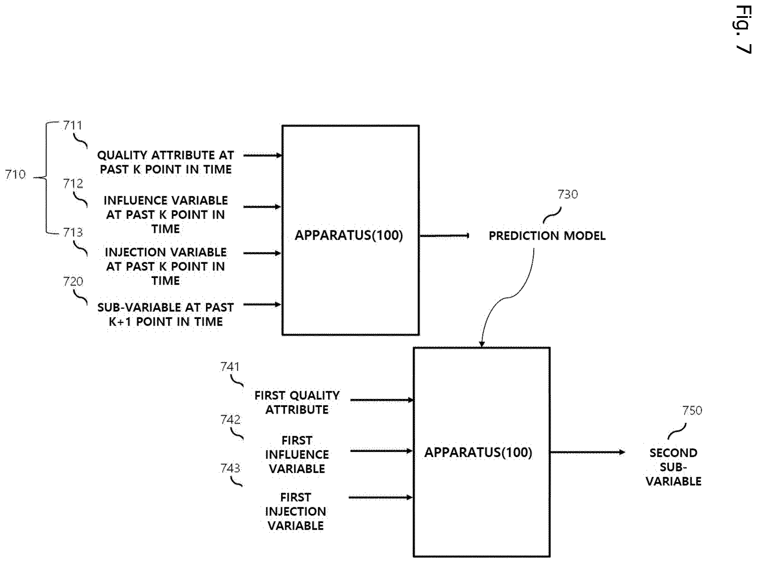

FIG. 7 is a block diagram for describing a prediction model according to an embodiment of the present invention.

The culture simulation model may include at least one of the prediction model for the changed amount of glutamine, the prediction model for the changed amount of glutamate, the prediction model for the consumed amount of glucose per VCD, the prediction model for the changed amount of lactate, the prediction model for the changed amount of ammonia, the prediction model for osmolarity, the prediction model for the cell division rate, the prediction model for the changed amount of viability, and the prediction model for the amount of IgG produced per VCD.

A prediction model 730 of FIG. 7 may include at least one of the prediction model for the changed amount of glutamine, the prediction model for the changed amount of glutamate, the prediction model for the consumed amount of glucose per VCD, the prediction model for the changed amount of lactate, the prediction model for the changed amount of ammonia, the prediction model for osmolarity, the prediction model for the cell division rate, the prediction model for the changed amount of viability, and the prediction model for the amount of IgG produced per VCD. The prediction model for the changed amount of glutamine, the prediction model for the changed amount of glutamate, the prediction model for the consumed amount of glucose per VCD, the prediction model for the changed amount of lactate, the prediction model for the changed amount of ammonia, the prediction model for osmolarity, the prediction model for the cell division rate, the prediction model for the changed amount of viability, and the prediction model for the amount of IgG produced per VCD, which are included in the culture simulation model, can be applied with the same description, and therefore are collectively referred to as the prediction model 730 in FIG. 7.

The apparatus 100 may acquire the prediction model 730 that machine-learns the correlation between the data 710 at the past k point in time and the sub-variable 720 at the past k+1 point in time. The machine learning algorithm for generating the machine learning prediction model 730 may include latent variable modeling including at least one of a neural network, a support vector machine, or partial least squares analysis. In addition, the machine learning prediction model 730 may be generated using the ensemble prediction model, and the machine learning algorithm for generating the ensemble prediction model may use the decision-making tree and the linear discriminant analysis including the NGBoost model or XGBoost model. The sub-prediction model included in the culture simulation model may be generated using an ensemble prediction model. That is, at least one of the prediction model for the changed amount of glutamine, the prediction model for the changed amount of glutamate, the prediction model for the consumed amount of glucose per VCD, the prediction model for the changed amount of lactate, the prediction model for the changed amount of ammonia, the prediction model for the osmolarity, the prediction model for the cell division rate, the prediction model for the changed amount of viability, and the prediction model for the amount of IgG produced per VCD may be generated using the ensemble prediction model.

In addition, the machine learning algorithm may use a reduced order time varying autoregressive exogenous model (ARX model). The process of acquiring the prediction model 730 from the data 710 at the past k point in time and the sub-variable 720 at the past k+1 point in time may be performed in the data learning unit 110 of the apparatus 100.

The data 710 at the past k point in time and the sub-variable 720 at the past k+1 point in time may be acquired based on data measured in real time through a Raman spectrometer. The k+1 point in time may be after the k point in time. The data 710 at the past k point in time may include at least one of a quality attribute 711 at the past k point in time, an influence variable 712 at the past k point in time, and an injection variable 713 at the past k point in time. The quality attribute 711 at the past k point in time, the influence variable 712 at the past k point in time, and the injection variables 713 at the past k point in time may be data sets for machine learning. The quality attribute, the influence variable, and the injection variable have already been described, and therefore detailed description thereof will be omitted.

In addition, the sub-variable at the past k+1 point in time may be label information corresponding to the data set. That is, the sub-variable 720 at the past k+1 point in time may be ground truth information. The sub-variable 720 at the past k+1 point in time may vary depending on the type of prediction model 730. In the case of the prediction model for the changed amount of glutamine, the sub-variable 720 at the k+1 point in time may be the amount of glutamine at the k+1 point in time. In the case of the prediction model for the changed amount of glutamate, the sub-variable 720 at the k+1 point in time may be the amount of glutamate at the k+1 point in time. In the case of the prediction model for the consumed amount of glucose per VCD, the sub-variable 720 at the k+1 point in time may be the consumed amount of glucose at the k+1 point in time. In the case of the prediction model for the changed amount of lactate, the sub-variable 720 at the k+1 point in time may be the amount of lactate at the k+1 point in time. In the case of the prediction model for the changed amount of ammonia, the sub-variable 720 at the k+1 point in time may be the amount of ammonia at the k+1 point in time. In the case of the prediction model for the changed amount of glutamate, the sub-variable 720 at the k+1 point in time may be the amount of glutamate at the k+1 point in time. In the case of the prediction model for the cell division rate, the sub-variable 720 at the k+1 point in time may be the cell division rate at the k+1 point in time. In the case of the prediction model for the changed amount of viability, the sub-variable 720 at the k+1 point in time may be the cell death rate at the k+1 point in time. In the case of the prediction model for the amount of IgG produced per VCD, the sub-variable 720 at the k+2 point in time may be IgG per VCD at the k+1 point in time.

The apparatus 100 may store the generated prediction model 730 or transmit the generated prediction model 730 to other devices. The apparatus 100 may receive the prediction model 730 from an external device.

The apparatus 100 may acquire the second sub-variable using the prediction model 730. The process of obtaining the second sub-variable 750 by applying at least one of the first quality attribute 741, the first influence variable 742, and the first injection variable 743 to the prediction model 730 to acquire the second sub-variable 750 may be performed in the data recognition unit 120 of the apparatus 100.

The first quality attribute 741, the first influence variable 742, and the first injection variable 743 may be values at the first point in time. The second sub-variable 750 may be the value at the second point in time. The first point in time may be before the second point in time. The difference between the first point in time and the second point in time may be 10 minutes, 1 hour, or 1 day, etc. The second sub-variable 750 may be a value predicted by the prediction model 730. Therefore, the second sub-variable 750 may be somewhat different from the ground truth information.

The second sub-variable 750 may vary depending on the type of prediction model 730. In the case of the prediction model for the changed amount of glutamine, the second sub-variable 750 may be the predicted amount of glutamine at the second point in time. In the case of the prediction model for the changed amount of glutamine, the second sub-variable 750 may be the predicted amount of glutamine at the second point in time. In the case of the prediction model for the consumed amount of glucose per VCD, the second sub-variable 750 may be the predicted consumed amount of glucose at the second point in time. In the case of the prediction model for the changed amount of lactate, the second sub-variable 750 may be the predicted amount of lactate at the second point in time. In the case of the prediction model for the changed amount of ammonia, the second sub-variable 750 may be the predicted amount of ammonia at the second point in time. In the case of prediction model for osmolarity, the second sub-variable 750 may be the predicted osmolarity at the second point in time. In the case of the prediction model for the cell division rate, the second sub-variable 750 may be the predicted cell division rate at the second point in time. In the case of the prediction model for the changed amount of glutamine, the second sub-variable 750 may be the predicted cell death rate at the second point in time. In the case of the prediction model for the amount of IgG produced per VCD, the second sub-variable 750 may be the predicted IgG per VCD at the second point in time. At least one of the above prediction models may be combined to form the culture simulation model.

FIG. 8 is a diagram for describing the predetermined condition according to the embodiment of the present invention. FIG. 9 is a diagram for describing a predetermined condition according to an embodiment of the present invention. FIG. 10 is a diagram for describing a predetermined condition according to an embodiment of the present invention. FIG. 11 is a diagram for describing a predetermined condition according to an embodiment of the present invention.

Within a first period, the apparatus 100 may change the first injection variable so that a second VCD value included in the second quality attribute has a maximum value. In addition, within a second period, the apparatus 100 may change the first injection variable so that the second amount of IgG value included in the second quality attribute has a maximum value.

The apparatus 100 may perform a test on the plurality of first injection variables in order to change the first injection variable. In addition, performing the test may mean acquiring the predicted second influence variable and the predicted second quality attribute for each of the plurality of first injection variables. The apparatus 100 may select the first injection variable that meets the predetermined condition among the plurality of first injection variables. The predetermined condition may be that the second VCD value included in the predicted second quality attribute has the maximum value or the second amount of IgG has the maximum value. The selected first injection variable may be the first injection variable that has been changed. Hereinafter, the operation of the apparatus 100 will be described in more detail.

Referring to FIG. 8, the apparatus 100 may select the influence variable or the quality attribute that meets the predetermined condition among the plurality of influence variables or the plurality of quality attributes, and select the injection variable corresponding to the selected influence variable or the quality attribute, thereby determining the optimal injection variable. In this way, the apparatus 100 may set an objective function to select the optimal first injection variable among the plurality of first injection variables. The objective function may be the influence variable or the condition that the quality attribute should meet. For example, the objective function may be defined as the case where an influence variable does not exceed the threshold or the quality attribute has the maximum value. For example, the objective function may be a function that selects the maximum value among the plurality of second VCD values. In addition, the obj ective function may be a function that selects the maximum amount among the plurality of second amounts of IgG. In addition, the objective function may be a function that selects the maximum value among the plurality of second VCD values in the first period of culture, and may be a function that selects the maximum value among the plurality of second amounts of IgG in the second period. The first period may be before the second period. For example, the first period may be day 4 or more but less than day 8, and the second period may be day 8 or more. No particular control may be performed on the feed before the first period. That is, the objective function may be as illustrated in FIG. 8. In FIG. 8, VCD_{predicted} may correspond to the plurality of second VCD values. Also, in FIG. 8, IgG_{predicted} may correspond to the plurality of second amounts of IgG.

When the objective function is as illustrated in FIG. 8, the apparatus 100 may further perform the following operations to perform the operation 340 of selecting the second influence variable and the second quality attribute. Within the first period, the apparatus 100 may perform an operation of selecting the maximum value among the plurality of second VCD values included in the plurality of second quality attributes as the second quality attribute. In addition, within the second period, the apparatus 100 may perform an operation of selecting the maximum value among the plurality of second VCD values included in the plurality of second quality attributes as the second quality attribute. The first period may be a predetermined range of time during which the cells are cultured in the cell culture medium. The second period may be after the first period. The second period may not overlap the first period.

In addition, the first point in time may be a point in time that has a measured value. In addition, the second point in time is a point in time that does not have a measured value, but is a point in time when the predicted value may be acquired based on the measured value at the first point in time. The second point in time may be after a predetermined time has elapsed from the first point in time. The predetermined time may be 10 minutes, 1 hour, 1 day, etc. In addition, the first point in time is a point in time when the measured value may be measured, and thus, may change over time. For example, the first point in time may be the present. The second point in time is after the predetermined time has passed from the first point in time, and as the first point in time changes, the second point in time may also change.

Both the first period and the second period may include the first point in time and the second point in time. That is, within the first period, the apparatus 100 may acquire the measured value (first point in time) and then acquire the predicted value at the second point in time after the predetermined time has passed from the first point in time based on the measured value. In addition, within the second period, the apparatus 100 may acquire the measured value (first point in time) and then acquire the predicted value at the second point in time after the predetermined time has passed from the first point in time based on the measured value.

The apparatus 100 may acquire the predicted value at the second point in time (future point in time) based on the measured value at the first point in time (current) included in the first period. The measured value may be the first influence variable or the first quality attribute. In addition, the predicted value may be the plurality of second influence variables or the plurality of second quality attributes. The apparatus 100 may select the maximum value among the plurality of second VCD values for the predicted value in the first period. In addition, the apparatus 100 may select the maximum value among the plurality of second amounts of IgG for the predicted value in the second period.

In addition, the apparatus 100 may perform the operation 350 of selecting the first injection variable corresponding to the selected second quality attribute among the plurality of first injection variables. In addition, the apparatus 100 may determine the selected first injection variable as the optimal injection variable and transmit the optimal injection variable to the bioreactor. In addition, the bioreactor may determine the culturing conditions for the first point in time based on the received optimal injection variable. Through this process, the bioreactor may produce a maximum amount of antibodies.

Referring to FIG. 9, the user may wish to minimize the amount of ammonia while meeting the conditions of FIG. 8. Within the first period, the apparatus 100 may perform an operation of changing the first injection variable so that the value obtained by multiplying the second VCD value included in the second quality attribute by e^ (-k* second amount of ammonia) has the maximum value. In addition, within the second period, the apparatus 100 may perform an operation of changing the first injection variable so that the value obtained by multiplying the second amount of IgG included in the second quality attribute by e^ (-k* second amount of ammonia) has the maximum value. The apparatus 100 may perform a test on the plurality of first injection variables in order to change the first injection variable. In addition, performing the test may mean acquiring the predicted second influence variable and the predicted second quality attribute for each of the plurality of first injection variables. The apparatus 100 may select the first injection variable that meets the predetermined condition among the plurality of first injection variables. The predetermined condition may mean that the value obtained by multiplying the second VCD included in the predicted second quality attribute multiplied by e^(-k* second amount of ammonia) has the maximum value or the value obtained by multiplying the second amount of IgG by e^(-k* second amount of ammonia) has the maximum value. The selected first injection variable may be the first injection variable that has been changed. Hereinafter, the operation of the apparatus 100 will be described in more detail.

Referring to FIG. 9, the user may wish to minimize the amount of ammonia while meeting the conditions of FIG. 8. In this case, the objective function may be as illustrated in FIG. 9. In FIG. 9, Ammonia_{predicted} may correspond to the plurality of second amounts of ammonia. The apparatus 100 may maintain *VCD_{predicted}* × *e*^{-0.1 *Ammonia*_{predicted}} at the maximum or maintain *IgG_{predicted}* × *e*^{-0.1 *Ammonia*_{predicted}} at the minimum to maintain the second amount of ammonia at a minimum while maximizing the VCD value or the amount of IgG.

The plurality of first injection variables may correspond one-to-one to the plurality of second VCD values. In addition, the plurality of first injection variables may correspond one-to-one to the plurality of second amounts of IgG. In addition, the plurality of first injection variables may correspond one-to-one to the plurality of second amounts of ammonia. The apparatus 100 may substitute one of the plurality of second VCD values and the second amount of ammonia corresponding to the one second VCD value into the equation of FIG. 9. In addition, the apparatus 100 may substitute one of the plurality of second amounts of IgG and the second amount of ammonia corresponding to one second amount of IgG into the equation of FIG. 9.

The apparatus 100 may further perform the following processes to perform the operation 340 of selecting the second influence variable and the second quality attribute. Referring to FIG. 9, within the first period, the apparatus 100 may perform an operation of selecting, as the second quality attribute, the VCD value when the value obtained by multiplying the plurality of second VCD values included in the plurality of second quality attributes by e^(-k*second amount of ammonia) is the maximum value. For example, the first period may be day 4 or more but less than day 8. In addition, k may be a positive number. For example, k may have a value exceeding 0 and less than or equal to 1. For example, k may be 0.1. In addition, within the first period, the apparatus 100 may perform an operation of selecting, as the second quality attribute, the second amounts of IgG when the value obtained by multiplying the plurality of second amounts of IgG included in the plurality of second quality attributes by e^(-k*second amount of ammonia) is the maximum value. In addition, k may be a positive number. For example, k may have a value exceeding 0 and less than or equal to 1. For example, k may be 0.1.

Since e^(-k * second amount of ammonia) decreases as the second amount of ammonia increases and increases as the second amount of ammonia decreases, the second amount of ammonia may be minimized while the second VCD value or the second amount of IgG becomes maximum so that the value obtained by multiplying the plurality of second VCDs by e^(-k * second amount of ammonia) or the value obtained by multiplying the plurality of second amounts of IgG by e^(-k* second amount of ammonia) becomes the maximum value.

In addition, the apparatus 100 may perform the operation 350 of selecting the first injection variable corresponding to the selected second quality attribute among the plurality of first injection variables. In addition, the apparatus 100 may determine the selected first injection variable as the optimal injection variable and transmit the optimal injection variable to the bioreactor 1810. In addition, the bioreactor 1810 may determine the culturing conditions for the first point in time based on the received optimal injection variable. Through this process, the bioreactor 1810 may produce a maximum amount of antibodies.

Referring to FIG. 10, a user may wish to limit the amount of lactate to less than or equal to a first threshold amount while meeting the conditions of FIG. 8 or FIG. 9. The apparatus 100 may perform the operation of determining whether the second amount of lactate included in the second influence variable is less than or equal to the first threshold amount. The apparatus 100 may perform an operation of changing the first injection variable so that the second VCD value included in the second quality attribute has the maximum value when the second amount of lactate is less than or equal to the first threshold amount and within the first period. In addition, the apparatus 100 may perform the operation of changing the first injection variable so that the second amount of IgG included in the second quality attribute has the maximum value when the second amount of lactate is less than or equal to the first threshold amount and within the second period. The apparatus 100 may perform a test on the plurality of first injection variables in order to change the first injection variable. In addition, performing the test may mean acquiring the predicted second influence variable and the predicted second quality attribute for each of the plurality of first injection variables. The apparatus 100 may select the first injection variable that meets the predetermined condition among the plurality of first injection variables. The predetermined condition may mean that the second amount of lactate included in the predicted second influence variable is less than or equal to the first threshold amount and the second VCD value included in the predicted second quality attribute has the maximum value, or the second amount of lactate included in the predicted second influence variable is less than or equal to the first threshold amount and the second amount of IgG included in the predicted second quality attribute has the maximum value. The selected first injection variable may be the first injection variable that has been changed. Hereinafter, the operation of the apparatus 100 will be described in more detail.

Referring to FIG. 10, a user may wish to limit the amount of lactate to less than or equal to the first threshold amount while meeting the conditions of FIG. 8 or FIG. 9. In this case, the objective function may be as illustrated in FIG. 10. The first threshold amount may be 1.2 g/L. When the objective function is set in this way, the apparatus 100 may derive the optimal results as long as the amount of lactate included in the predicted influence variable does not exceed the first threshold amount. More specifically, when the predicted amount of lactate exceeds the first threshold amount, the apparatus 100 may set the predicted corresponding second VCD value or the second predicted amount of IgG corresponding to the predicted amount of lactate to 0. Accordingly, when the predicted amount of lactate exceeds the first threshold amount, the predicted corresponding second VCD value or the predicted corresponding second amount of IgG may not be selected as the maximum value.

In FIG. 10, when the amount of lactate included in the predicted influence variable exceeds the first threshold amount, the apparatus 100 describes a configuration for setting the predicted corresponding second VCD value or the predicted second corresponding amount of IgG to 0, but is not limited thereto. When the amount of lactate included in the predicted influence variable exceeds the first threshold amount, the apparatus 100 may determine the predicted corresponding second VCD value or the predicted second corresponding amount of IgG as a value obtained by multiplying the predicted second VCD value or the predicted second amount of IgG by a small positive number less than or equal to 0.01. Accordingly, the predicted corresponding second VCD value or the predicted amount of corresponding second IgG may not be selected as the maximum value.

More specifically, the apparatus 100 may remove the injection variable in which the amount of lactate exceeds the first threshold amount for day 1 or more and day 3 or less of culture. Alternatively, the apparatus 100 may not control the injection variable for day 1 or more and day 3 or less of culture. In addition, the measurement may not be performed on the influence variable or the quality attribute. That is, the apparatus 100 may perform control from the first period.

Lactate_{predicted} may correspond to the plurality of second amounts of lactate. The apparatus 100 may remove the second quality attribute, the second influence variable, and the first injection variable corresponding to the second amount of lactate exceeding the first threshold amount. In addition, the apparatus 100 may determine the optimal first injection variable using some second quality attributes, some second influence variables, and some first injection variables corresponding to the second amount of lactate less than or equal to the first threshold amount.

More specifically, the apparatus 100 may further perform the following process when performing the operation 340 of selecting the second influence variable and the second quality attribute. The apparatus 100 may perform an operation of acquiring some second influence variables in which the plurality of second amounts of lactate among the plurality of second influence variables is less than or equal to the first threshold amount. In addition, the apparatus 100 may perform an operation of acquiring some second quality attributes corresponding to some second influence variables. In addition, within the first period, the apparatus 100 may perform an operation of selecting the maximum value among the plurality of second VCD values included in some second quality attributes as the second quality attribute. In addition, within the second period, the apparatus 100 may perform an operation of selecting the maximum value among the plurality of second amounts of IgG included in some second quality attributes as the second quality attribute.

The apparatus 100 may perform the operation 350 of selecting the first injection variable corresponding to the selected second quality attribute among the plurality of first injection variables. The apparatus 100 may perform the operation of determining the first injection variable selected in operation 350 as the optimal injection variable at the first point in time. In addition, the apparatus 100 may determine the selected first injection variable as the optimal injection variable and transmit the optimal injection variable to the bioreactor 1810. In addition, the bioreactor 1810 may also determine culturing conditions for the first point in time based on the received optimal input variable. Through this process, the bioreactor 1810 may produce a maximum amount of antibodies.

Referring to FIG. 11, a user may wish to limit the amount of glutamate to greater than or equal to the second threshold amount and less than or equal to a third threshold amount while meeting one condition of FIGS. 8 to 10. The apparatus 100 may perform an operation of determining whether the second amount of glutamate included in the second influence variable is greater than or equal to the second threshold amount and less than or equal to the third threshold amount. The apparatus 100 may perform an operation of changing the first injection variable so that the second VCD value included in the second quality attribute has the maximum value when the second amount of glutamate is greater than or equal to the second threshold and less than or equal to the third threshold and within the first period. The apparatus 100 may perform the operation of changing the first injection variable so that the second amount of IgG included in the second quality attribute has the maximum value when the second amount of glutamate is greater than or equal to the second threshold amount and less than or equal to the third threshold amount and within the second period. The apparatus 100 may perform a test on the plurality of first injection variables in order to change the first injection variable. In addition, performing the test may mean acquiring the predicted second influence variable and the predicted second quality attribute for each of the plurality of first injection variables. The apparatus 100 may select the first injection variable that meets the predetermined condition among the plurality of first injection variables. The predetermined condition may mean that the second VCD value included in the predicted second quality attribute has the maximum value while the second amount of glutamate included in the predicted second influence information is greater than or equal to the second threshold amount and less than or equal to the third threshold amount or the second amount of glutamate included in the predicted second influence variable is greater than or equal to the second threshold amount and less than or equal to the third threshold amount, and the second amount of IgG included in the second quality attribute has the maximum value. The selected first injection variable may be the first injection variable that has been changed. The changed first injection variable may be the optimal first injection variable. Hereinafter, the operation of the apparatus 100 will be described in more detail.

Referring to FIG. 11, a user may wish to limit the amount of glutamate to greater than or equal to the second threshold amount and less than or equal to a third threshold amount while meeting one condition of FIGS. 8 to 10. For example, the second threshold amount may be 0.45 g/L. In addition, the third threshold amount may be 0.65 g/L. In this case, the objective function may be as illustrated in FIG. 11. When the objective function is set in this way, the apparatus 100 may derive the optimal results as long as the amount of glutamate is less than the second threshold amount or does not exceed the third threshold amount. More specifically, when the predicted amount of glutamate is less than the second threshold amount and exceeds the third threshold amount, the apparatus 100 may set the predicted corresponding second VCD value or the predicted amount of corresponding second IgG, corresponding to the predicted amount of lactate, to 0. Accordingly, when the predicted amount of lactate is less than the second threshold amount and exceeds the third threshold amount, the predicted corresponding second VCD value or the predicted amount of corresponding second IgG may not be selected as the maximum value.

FIG. 11 shows a configuration in which the apparatus 100 sets the predicted second VCD value or the second amount of IgG to 0 when the amount of glutamate included in the predicted influence variable is less than the second threshold amount and exceeds the third threshold amount, but it is not limited thereto. When the amount of glutamate included in the predicted influence variable is less than the second threshold amount and exceeds the third threshold amount, the apparatus 100 may determine the predicted corresponding second VCD value or the predicted second corresponding amount of IgG as a value obtained by multiplying the predicted corresponding second VCD value or the predicted second corresponding amount of IgG by a small positive number less than or equal to 0.01. Accordingly, the predicted corresponding second VCD value or the predicted second corresponding amount of IgG may not be selected as the maximum value.

More specifically, the apparatus 100 may remove the injection variable in which the amount of glutamate is less than the second threshold amount or exceeds the third threshold amount for day 1 or more and day 3 or less of culture. Alternatively, the apparatus 100 may not control the injection variables for day 1 or more and day 3 or less of culture. In addition, the measurement may not be performed on the influence variable or the quality attribute. That is, the apparatus 100 may perform control from the first period.

Glutamate_{predicted} may correspond to the plurality of second amounts of glutamate. The apparatus 100 may remove the second quality attribute, the second influence variable, and the first injection variable corresponding to the second amount of glutamate that is less than the second threshold amount or exceeds the third threshold amount. In addition, the apparatus 100 may determine the optimal first injection variable using some second quality attributes, some second influence variables, and some first injection variables corresponding to the second amount of glutamate that is greater than or equal to the second threshold amount and less than or equal to the third threshold amount.

More specifically, the apparatus 100 may further perform the following processes when performing the operation 340 of selecting the second influence variable and the second quality attribute. The apparatus 100 may perform an operation of acquiring some second influence variables in which a plurality of second glutamate concentrations are greater than or equal to the second threshold amount and less than or equal to the third threshold amount among the plurality of second influence variables. In addition, the apparatus 100 may perform an operation of acquiring some second quality attributes corresponding to some second influence variables. In addition, within the first period, the apparatus 100 may perform the operation of selecting the maximum value among the plurality of second VCD values included in some second quality attributes as the second quality attribute. In addition, within the second period, the apparatus 100 may perform the operation of selecting the maximum value among the plurality of second amounts of IgG included in some second quality attributes as the second quality attribute.

The apparatus 100 may perform the operation 350 of selecting the first injection variable corresponding to the selected second quality attribute among the plurality of first injection variables. The apparatus 100 may perform the operation of determining the first injection variable selected in operation 350 as the optimal injection variable at the first point in time. In addition, the apparatus 100 may determine the selected first injection variable as the optimal injection variable and transmit the optimal injection variable to the bioreactor 1810. In addition, the bioreactor 1810 may determine the culturing conditions for the first point in time based on the received optimal injection variable. Through this process, the bioreactor 1810 may produce a maximum amount of antibodies.

The apparatus 100 may select the optimal first injection variable for the second point in time in operation 350 of FIG. 3. In addition, the apparatus 100 may predict a third influence variable and a third quality attribute based on the second influence variable and the second quality attribute at the second point in time. The apparatus 100 may predict the third influence variable and the third quality attribute using the second influence variable and the second quality attribute at the second point in time predicted in FIG. 3. The third influence variable and the third quality attribute may be predicted information at the third point in time. The third point in time may be a future of the second point in time. The interval between the first point in time and the second point in time may be the same as the interval between the second point in time and the third point in time.

However, the apparatus 100 is not limited to predicting the third influence variable and the third quality attribute using the predicted second influence variable and second quality attribute at the second point in time predicted in FIG. 3. The apparatus 100 may measure the actual second influence variable and the actual second quality attribute at the second point in time, and predict the third influence variable and the third quality attribute based on the actual second influence variable and the actual second quality attribute. This is because actual measurement is possible when the second point in time becomes the current point in time.

More specifically, the apparatus 100 may perform an operation of acquiring the plurality of second injection variables included within a predetermined range from the selected first injection variable. The operation of acquiring the plurality of second injection variables may correspond to the operation of acquiring the plurality of first injection variables.

The apparatus 100 may perform an operation of applying the selected second quality attribute, the selected second influence variable, and the plurality of second injection variables to the culture simulation model to acquire a plurality of third quality attributes and a plurality of third influence variables. The operation of acquiring the plurality of third quality attributes and the plurality of third influence variables may correspond to operation 330. The selected second quality attribute and the selected second influence variable may be the second quality attribute or the second influence variable predicted and selected in FIG. 3. In addition, the selected second quality attribute and the selected second influence variable may be the second quality attribute or the second influence variable at a second point in time that is actually measured.

The apparatus 100 may perform an operation of selecting the third quality attribute and the third influence variable that meet the predetermined condition among the plurality of third quality attributes and the plurality of third influence variables. The operation of selecting the third quality attribute and the third influence variable may correspond to operation 340.

The apparatus 100 may perform the operation of selecting the second injection variable corresponding to the selected third quality attribute among the plurality of first injection variables. The operation of selecting the second injection variable may correspond to operation 350 of FIG. 3. The apparatus 100 may repeatedly perform the processes to acquire predicted influence variables and quality attributes further in the future. More specifically, the apparatus 100 may acquire a predicted fourth quality attribute and a predicted fourth influence variable based on the selected third quality attribute, the selected third influence variable, and the third injection variables. As already described, the time between the first point in time and the second point in time and the time between the second point in time and the third point in time may be 10 minutes, 1 hour, or 1 day. The apparatus 100 may predict the quality attribute or the influence variables further in the future by performing predictions by each unit of time. For example, the apparatus 100 may predict the quality attribute or the influence variable after 4 days or after a week.

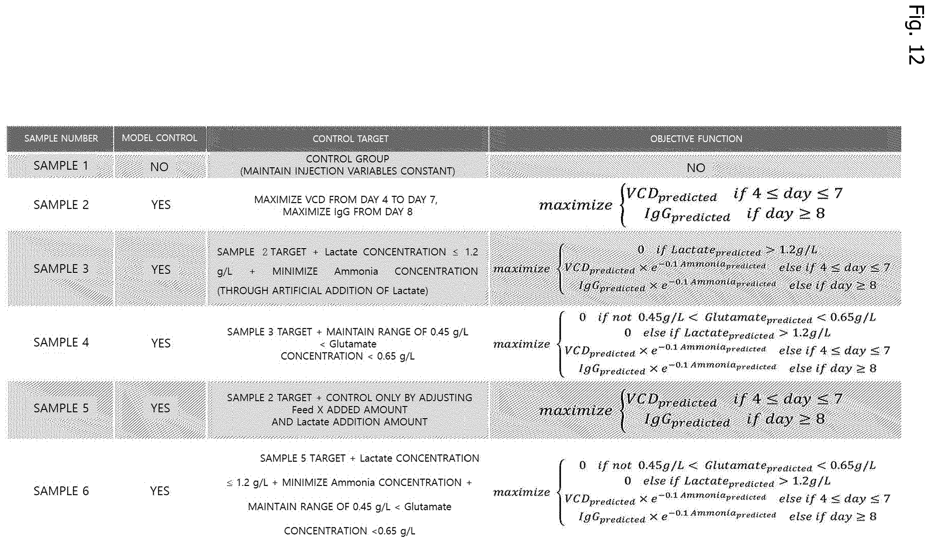

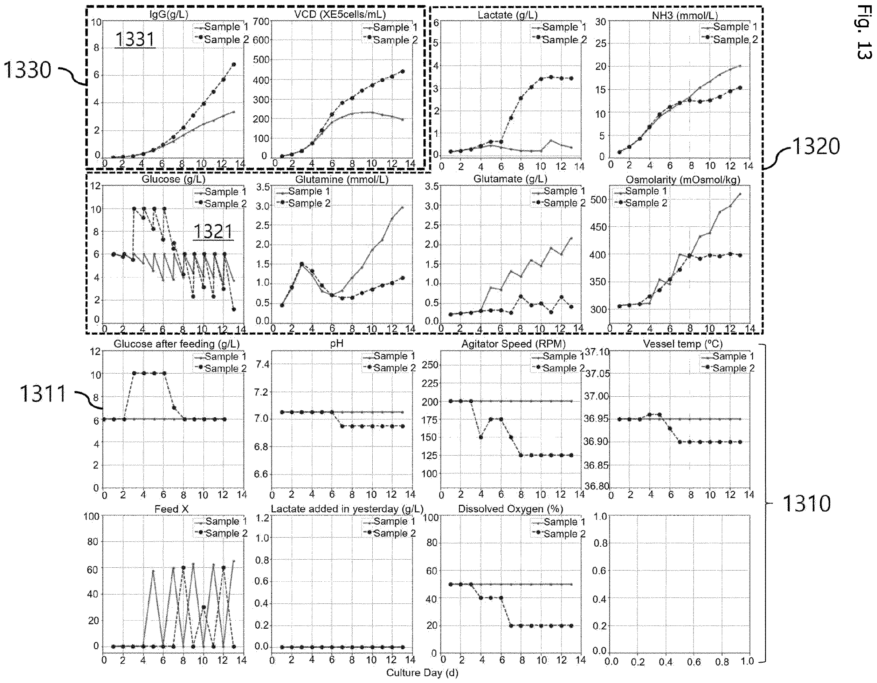

FIG. 12 is a table illustrating a simulation condition according to an embodiment of the present invention. In addition, FIGS. 13 to 17 may be simulation results according to an embodiment of the present invention.

All six samples of the embodiments in FIGS. 13 to 17 are the results of a simulation test of a virtual CHO cell culture and antibody production experiment with the control target disclosed in FIG. 12 from day 0 to day 13.

Glucose after feeding (g/L) in the embodiments in FIGS. 13 to 17 is the glucose concentration (g/L) measured immediately after glucose was added to the culture medium. That is, the apparatus 100 may inject glucose into the current feed based on the amount of glucose included in the injection variables selected in operation 350, and the glucose after feeding (g/L) may be the amount of glucose in the feed after the injection of glucose into the current batch based on the amount of glucose included in the selected injection variables.

The agitator speed (RPM) in the embodiments of FIGS. 13 to 17 indicates how many times the agitator rotates per minute.

Vessel temp (°C) in the embodiments of FIGS. 13 to 17 is the temperature within the bioreactor.

Feed X in the embodiments of FIGS. 13 to 17 refers to how much some kinds of nutrient feeds, which correspond to a nitrogen source, etc., for Chinese hamster ovary (CHO) cells were added to the culture medium in the bioreactor in the previous time unit.

Lactate added yesterday (g/L) in the embodiment of FIGS. 13 to 17 is how many grams of lactate were added to 1 L of culture medium in the previous time unit. When adding the lactate, the lactate is Ph-neutralized in advance with a basic substance and then added. The increase in osmolarity when 1 g of lactate pH-neutralized with a basic substance was added per 1 L of culture medium was arbitrarily set to 22 mOsmol/kg on the culture simulation system.

Dissolved oxygen (%) in the embodiments of FIGS. 13 to 17 is the dissolved oxygen (%) in the culture medium in the bioreactor.

FIG. 13 is a graph representing the simulation test results of sample 1 and sample 2.

Graphs 1310 related to injection variables in FIG. 13 represent the injection variables selected over time among the plurality of injection variables. In the graphs 1310, the first point in time may be a point in time immediately before the second point in time. For example, in the graphs 1310, when day 1 is the first point in time, day 2 may be the second point in time, and when day 2 is the first point in time, day 3 may be the second point in time.

Graphs 1320 related to injection variable in FIG. 13 may represent the injection variables selected over time among the plurality of injection variables. The graphs 1320 may be about the predicted second influence variable according to the selected first injection variable. For example, when the first amount of glucose included in the first injection variable selected on day 4 is 10 g/L (see reference numeral 1311), the second amount of glucose included in the second influence variable predicted on day 5 may be slightly less than about 9 g/L (see reference numeral 1321).

The quality attribute-related graphs 1330 of FIG. 13 may represent the quality attribute selected over time among the plurality of quality attributes. The graphs 1330 may be about the predicted second quality attribute according to the selected first injection variable. For example, when the first amount of glucose included in the first injection variable selected on day 4 is 10 g/L (see reference numeral 1311), the second amount of IgG included in the second influence variable predicted on day 5 may be slightly less than about 1 g/L (see reference numeral 1331).

Sample 1 is a result of a re-simulation test of actual CHO cell culture experiment data, not a simulation, after constantly fixing injection variables other than the Feed X. This is almost similar to the actual culture data of the corresponding experiment, and serves as a control group in the present embodiment.

Sample 2 is a result of a simulation-based control system being cultivated by setting a combination of injection variable values for each day to maximize only the VCD values of each day from day 4 to day 8 of culture (first period) and only the IgG concentrations of each day from day 8 of culture (second period). That is, the conditions may be the same as those in FIG. 8. Meanwhile, sample 2 may be a result of maximizing only the objective function of the second point in time (future point in time) which is the point in time following the first point in time (for example, the current point in time) and setting the combination of injection variable values.

The simulation-based control system adjusted the injection variables for each day of culture to maximize the objective function, resulting in a higher final IgG concentration than that of sample 1 which is a control group. Specifically, by adding a smaller amount of Feed X overall in sample 2 than in sample 1 which is the control group, the osmolarity, the glutamine concentration, and the glutamate concentration of the culture medium were generally maintained at lower values than the control group, and from day 7 of culture, the pH, the agitator speed (RPM), and the vessel temp (°C) were intentionally set lower than the control group. However, the simulation-based control system of the present invention showed a unique control tendency to intentionally lower the dissolved oxygen (%) to 20% from day 7 of culture and amplify the lactate concentration to about 3.4 g/L. Since the high lactate concentration tends to suppress the increase in ammonia concentration, in the simulation-based control system of the present invention, it is determined that suppressing the production of ammonia, which has a negative effect on the CHO cells, with the lactate generated through this process is more beneficial than setting the dissolved oxygen (%) to a high value in terms of the objective function from day 7 of culture.

However, because the prediction model used in the present embodiment has never trained similar culture data, it may be uncertain whether this control strategy will produce positive results like sample 2 in actual culture rather than simulation. In addition, a smaller amount of Feed X was added in sample 2 than in sample 1, but in actual culture, it may be unclear exactly what results lowering the added amount of Feed X will bring. However, when these inexperienced strategies are applied to actual culture, even if the result is more negative than sample 2 in FIG. 13, if the culture simulation system is further trained on the data of the negative results, the subsequent simulation-based control system may also perform further control with this result in mind, resulting in improving its own control performance and accuracy.

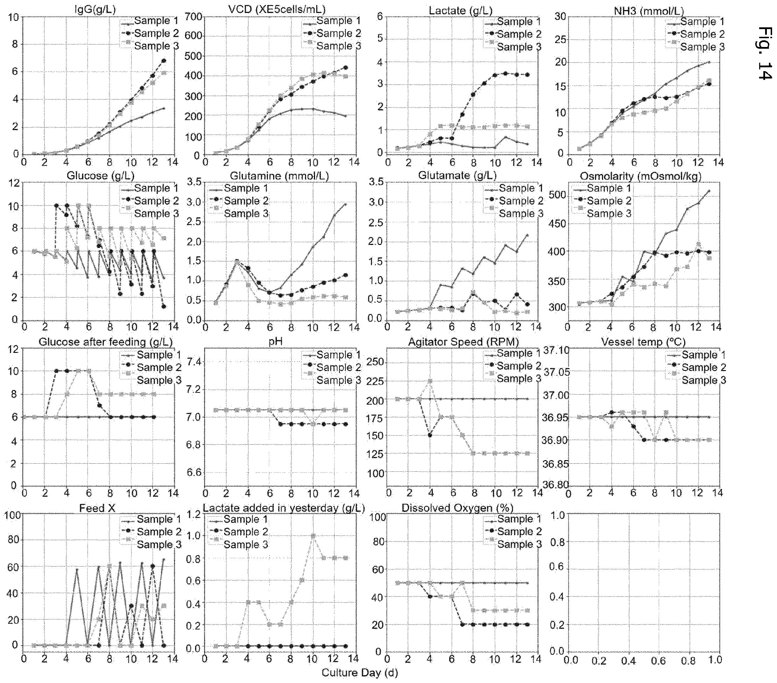

Similar to FIG. 13, FIG. 14 may include graphs related to injection variables, graphs related to influence variables, and graphs related to quality attributes.

FIG. 14 is a graph representing the simulation test results of sample 1, sample 2, and sample 3. Sample 1 and sample 2 are the same as described above. Sample 3 was basically the same as the control target of sample 2, but the objective function was set to maintain the amount of lactate less than or equal to 1.2 g/L and minimize the amount of ammonia. The objective function of sample 3 may be the same as that of FIG. 10. The objective function of sample 3 specifies that the objective function value is fixed to 0 when the predicted lactate concentration the next time unit exceeds 1.2 g/L, but when it is impossible to maintain the lactate concentration less than or equal to 1.2 g/L through the culture simulation system, all objective function values of which sizes need to be compared to each other become 0, making it impossible to derive actual optimal results. To prevent this, when the lactate concentration exceeds 1.2 g/L, instead of fixing the objective function to 0, alternatives such as using a value obtained by multiplying the predicted VCD value or the predicted amount of IgG as the objective function by a small positive number less than or equal to about 0.01 may be considered. Looking at the results of sample 3, in the apparatus 100 of the present invention, the lactate is intentionally added to the culture medium to set the dissolved oxygen (%) higher than that of sample 2, and even if the amount of IgG in the final primary culture is lower than sample 2, the ammonia concentration is also maintained lower than that of sample 2 from days 5 to 11 of culture while the lactate concentration is maintained within the range of 1.2 g/L or less during the entire culture period.

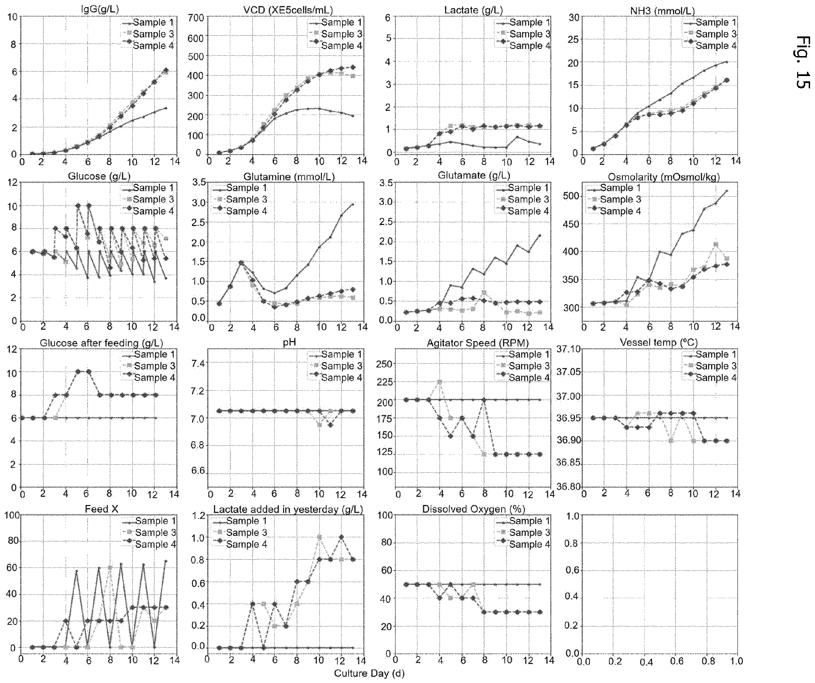

Similar to FIG. 13, FIG. 15 may include graphs related to injection variables, graphs related to influence variables, and graphs related to quality attributes.

FIG. 15 is a graph representing the simulation test results of sample 1, sample 3, and sample 4. Sample 1 and sample 3 are the same as described above. Sample 4 was basically the same as the control target of sample 3, but the objective function was set to control the glutamate concentration while maintaining the range of 0.45 g/L to 0.65 g/L from the 4 day or more of culture. The objective function of FIG. 15 may be the same as that of FIG. 11.

The apparatus 100 of the present invention achieved this target within the simulation by adjusting the added amount of Feed X. In addition, the IgG concentration in sample 4 on the final day of culture was calculated to be higher than that of sample 3. This may show that even if additional restrictions are placed on the objective function of the control system of the present invention, more optimal results may be produced from a long-term perspective.

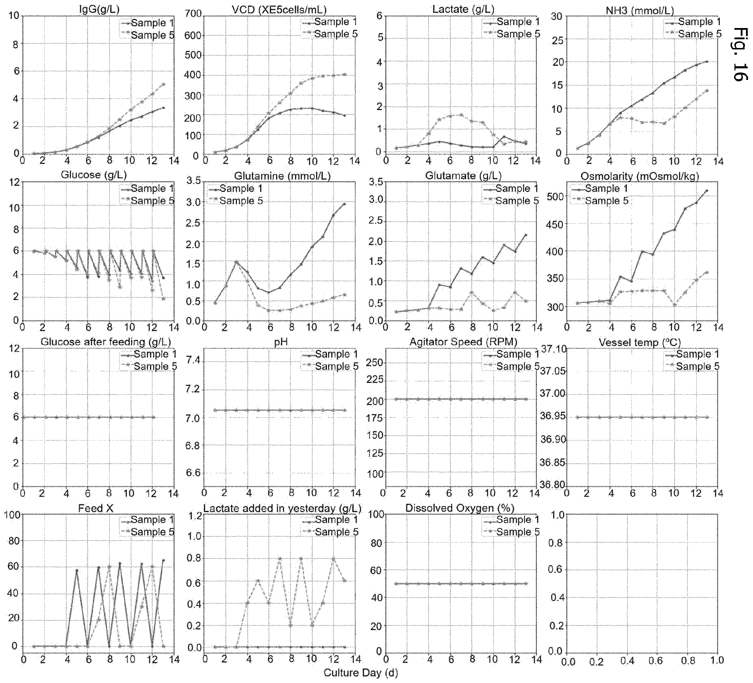

Similar to FIG. 13, FIG. 16 may include graphs related to injection variables, graphs related to influence variables, and graphs related to quality attributes.

FIG. 16 is a graph representing the simulation test results of sample 1 and sample 5. Sample 1 is the same as the description above. Sample 5 is basically the same as the control target of sample 2, but is the control result of keeping other injection variables constant as in sample 1 except the added amount of Feed X and the lactate added yesterday (g/L) among the injection variables during the culture. Sample 5 showed positive results of a final primary IgG concentration higher than that of sample 1. Sample 5 suppressed the increase in ammonia concentration by maintaining the lactate concentration of approximately 1.5 g/L during the culture, and maintained the osmolarity, the glutamine concentration, and the glutamate concentration at low values by adding a smaller amount of Feed X overall than in the sample 1 which is the control group. In this way, the user or the apparatus 100 may selectively modify at least one of the first amount of nutrient feed, the first amount of glucose in the nutrient medium, the first amount of oxygen molecules, the first amount of carbon dioxide molecules, the first amount of nitrogen molecules, the first amount of air, a first pH value, a first agitator speed, a first vessel temperature, a first media injection schedule, and the first amount of antifoam, thereby acquiring the optimal quality attribute.

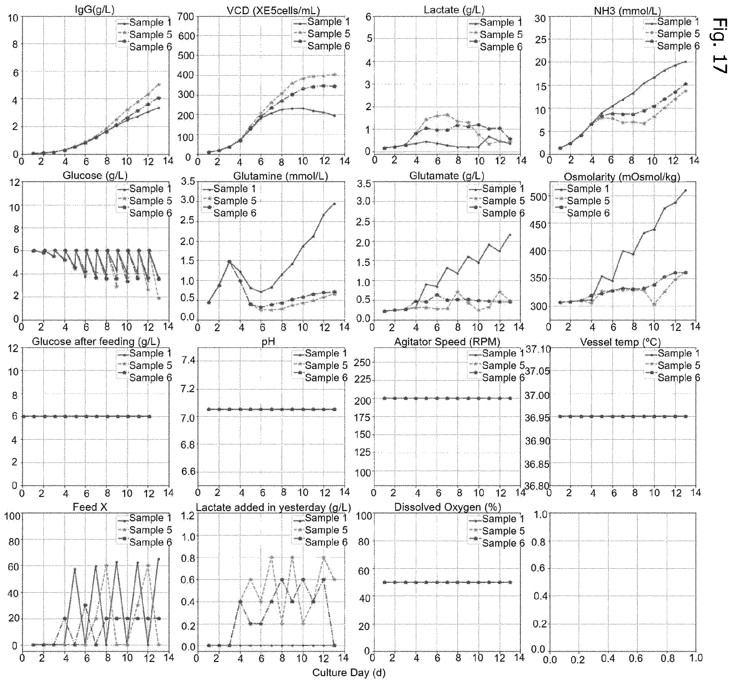

Similar to FIG. 13, FIG. 17 may include graphs related to injection variables, graphs related to influence variables, and graphs related to quality attributes.

FIG. 17 is a graph representing the simulation test results of sample 1, sample 5, and sample 6. Sample 1 and sample 5 are the same as described above. Sample 6 is basically the same as the control target of sample 5, but the objective function was set to control the lactate concentration to be maintained at 1.2 g/L or less, and the glutamate concentration to maintain the range of 0.45 g/L or more and 0.65 g/L or less from day 4 or more of culture. The lactate concentration and the glutamate concentration achieved the control target, the ammonia concentration was lower than sample 1 but higher than sample 5, and the IgG concentration at the final day of culture was also higher than sample 1 but lower than sample 5. However, FIG. 17 is significant in that it is theoretically possible for the simulation-based control system of the present invention to maximize or minimize the influence variables and the quality variables at the user's discretion, or maintain the influence variables and the quality variables within a desired range, only by adjusting the injection variables.

The apparatus 100 of the present invention predicts at least one of the influence variable and the quality attribute at the second point in time based on the injection variables, the influence variables, and the quality attributes at the first point in time. Here, the first point in time may be immediately before the second point in time. Therefore, the apparatus 100 pursues short-term results and may have a negative effect in the long term. Therefore, the apparatus 100 may determine the "optimal range" of the injection variable or influence variable that may lead to long-term optimal quality attributes by conducting research through various experiments or simulations. When only the optimal range of the injection variables or the influence variables is determined, it is fully possible to reset the objective function and control the injection variables or the influence variables to meet the optimal range using the simulation-based control system. In addition, the apparatus 100 may train AI reinforcement learning models such as a deep Q learning network to actively determine where to set the value range of the influence variable for each future point in time. By doing so, the apparatus 100 may determine the injection variables to obtain the optimal quality attributes from a long-term perspective.

So far, various embodiments have been mainly described. It will be understood by those skilled in the art to which the present invention pertains that the present invention may be implemented in a modified form without departing from essential characteristics of the present invention. Therefore, exemplary embodiments disclosed herein should be considered in an illustrative aspect rather than a restrictive aspect. The scope of the present invention should be defined by the claims rather than the above-mentioned description, and equivalents to the claims should be interpreted to fall within the present invention.

Meanwhile, the above-described embodiments of the present invention can be written as a program that may be executed on a computer, and can be implemented in a general-purpose digital computer that operates the program using a computer-readable recording medium. The computer-readable recording medium includes storage media such as magnetic storage media (e.g., a ROM, a floppy disk, a hard disk, etc.) and optically readable media (e.g., a compact disc read-only memory (CD-ROM), a digital video disc (DVD), etc.).

## Claims

1. A method of operating an apparatus for determining a cell culturing condition, comprising:
acquiring a first quality attribute and a first influence variable at a first point in time from a cell culture medium;
acquiring a first injection variable;
applying at least one of the first quality attribute, the first influence variable, and the first injection variable to a culture simulation model and acquiring a second quality attribute and a second influence variable at a second point in time; and
selectively changing the first injection variables so that the second quality attribute meets a predetermined condition,
wherein the second point in time is a future of the first point in time.

2. The method of claim 1, wherein the first quality attribute includes a first viable cell density (VCD) value and a first amount of immunoglobulin G (IgG).

3. The method of claim 1, wherein the first influence variable includes at least one of a first amount of glutamine, a first amount of glutamate, a first amount of glucose, a first amount of lactate, a first amount of ammonia, and first osmolarity.

4. The method of claim 3, wherein the acquiring of the first quality attribute and the first influence variable at the first point in time from the cell culture medium includes acquiring a plurality of measured first influence variables at the same point in time, and
the acquiring of the second quality attribute and the second influence variable at the second point in time includes acquiring a second influence variable predicted based on the plurality of measured first influence variables.

5. The method of claim 1, wherein the first injection variable includes at least one of a first amount of nutrient feed, a first amount of glucose in the nutrient medium, a first amount of oxygen molecules, a first amount of carbon dioxide molecules, a first amount of nitrogen molecules, a first amount of air, a first pH value, a first agitator speed, a first vessel temperature, a first media injection schedule, and a first amount of antifoam.

6. The method of claim 1, wherein the selectively changing of the first injection variable includes:
within a first period, changing the first injection variable so that a second viable cell density (VCD) value included in the second quality attribute has a maximum value; and
within a second period, changing the first injection variable so that a second amount of immunoglobulin G (IgG) included in the second quality attribute has the maximum value, and
the second period is after the first period.

7. The method of claim 6, wherein the selectively changing of the first injection variable includes:
within the first period, changing the first injection variable so that a value obtained by multiplying the second VCD value included in the second quality attribute by e^ (-k* second amount of ammonia) has the maximum value; and
within the second period, changing the first injection variable so that a value obtained by multiplying the second amount of IgG included in the second quality attribute by e^ (-k* second amount of ammonia) has the maximum value, and
the k is a positive number.

8. The method of claim 6, wherein the selectively changing of the first injection variable includes:
determining whether a second amount of lactate included in the second influence variable is less than or equal to a first threshold amount;
when the second amount of lactate is less than or equal to a first threshold amount and within the first period, changing the first injection variables so that the second VCD value included in the second quality attribute has the maximum value; and
when the second amount of lactate is less than or equal to the first threshold amount and within the second period, changing the first injection variable so that the second amount of IgG included in the second quality attribute has the maximum value.

9. The method of claim 6, wherein the selectively changing of the first injection variable includes:
determining whether a second amount of glutamate included in the second influence variable is greater than or equal to a second threshold amount and less than or equal to a third threshold amount;
when the second amount of glutamate is greater than or equal to the second threshold amount and less than or equal to the third threshold amount and within the first period, changing the first injection variables so that the second VCD value included in the second quality attribute has the maximum value; and
when the second amount of glutamate is greater than or equal to the second threshold amount and less than or equal to the third threshold amount and within the second period, changing the first injection variable so that the second amount of IgG included in the second quality attribute has the maximum value.

10. The method of claim 1, further comprising transmitting the changed first injection variable to a bioreactor.

11. An apparatus for determining a cell culturing condition, comprising:
a processor; and
a memory,
wherein, based on instructions stored in the memory, the processor acquires a first quality attribute and a first influence variable at a first point in time from a cell culture medium, acquires a first injection variable, applies at least one of the first quality attribute, the first influence variable, and the first injection variable to a culture simulation model to acquire a second quality attribute and a second influence variable at a second point in time, and selectively changes the first injection variables so that the second quality attribute meets a predetermined condition, and the second point in time is a future of the first point in time.
